(19) **Europäisches Patentamt / European Patent Office / Office européen des brevets**

(11) **EP 2 848 917 B1**

(12) # EUROPEAN PATENT SPECIFICATION

(45) Date of publication and mention
of the grant of the patent:
**20.02.2019 Bulletin 2019/08**

(21) Application number: **13787241.2**

(22) Date of filing: **08.05.2013**

(51) Int Cl.:
*G01N 21/25* ^(2006.01)    *G01N 33/28* ^(2006.01)
*G01N 21/85* ^(2006.01)    *B25J 19/00* ^(2006.01)
*B25J 19/02* ^(2006.01)    *F16N 29/00* ^(2006.01)
*G01N 21/53* ^(2006.01)

(86) International application number:
**PCT/JP2013/062940**

(87) International publication number:
**WO 2013/168741 (14.11.2013 Gazette 2013/46)**

(54) **LUBRICATING OIL DEGRADATION SENSOR AND MACHINE PROVIDED THEREWITH**

SCHMIERÖLVERSCHLEISSSENSOR UND DAMIT AUSGERÜSTETE MASCHINE

DÉTECTEUR DE DÉGRADATION D'HUILE DE LUBRIFICATION ET MACHINE ÉQUIPÉE DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2012 JP 2012108392**

(43) Date of publication of application:
**18.03.2015 Bulletin 2015/12**

(73) Proprietor: **Nabtesco Corporation
Tokyo 102-0093 (JP)**

(72) Inventors:
• **NAKAMURA, Koji
Tsu-shi, Mie 514-8533 (JP)**

• **SHIMADA, Hideshi
Tsu-shi, Mie 514-8533 (JP)**

(74) Representative: **Grünecker Patent- und Rechtsanwälte
PartG mbB
Leopoldstraße 4
80802 München (DE)**

(56) References cited:
WO-A1-2012/000539       JP-A- H05 118 986
JP-A- H06 281 575       JP-A- 2005 156 297
JP-A- 2007 192 769       JP-A- 2008 073 775
JP-A- 2010 071 733       US-A1- 2010 045 989
US-A1- 2011 249 257

## Description

Technical Field

The present invention relates to a lubricant deterioration sensor for detecting deterioration of lubricant of a machine.

Background Art

In the related art, as a lubricant deterioration sensor for detecting deterioration of lubricant of a machine, an oil deterioration degree sensor has been known. In this sensor, an oil entering gap portion which the lubricant is allowed to enter is formed on an optical path from an infrared a light emitting diode (LED) to a photo diode. Then, a light absorption quantity of the lubricant inside the oil entering gap portion with respect to light emitted from the infrared LED is measured according to a light reception quantity of the photo diode, thereby determining a deterioration degree of the lubricant related to the measured light absorption quantity (for example, refer to PTL 1 and PTL 2).

However, the oil deterioration degree sensor described in PTL 1 and PTL 2 can measure the density of insoluble content contained in the lubricant as the deterioration degree of the lubricant, but has a problem in that types of contaminant contained in the lubricant cannot be specified.

As a technique of identifying the types of contaminant contained in the lubricant, a technique has been known in which an LED emits light toward a membrane filter which has filtered the lubricant. Then, a light receiving element converts reflection light from the contaminant on the membrane filter into an RGB digital value, thereby identifying the types of contaminant contained in the lubricant, based on the converted RGB digital value (for example, refer to NPL 1 and NPL 2). Patent document US 2010/045989 A1 relates to a method for monitoring the condition of a medium in a channel, based on the transmission/emission of light in which the condition of the medium is evaluated, using measuring electronics. Patent document US 2011/249257 A1 relates to an optical sensor array for detecting a first liquid medium in a second liquid medium by means of the reflection of an emitted light beam of a given wavelength, comprising a light source and an associated receiver, thereby determining quantitatively free water in fuels and the presence of water in containers.

Citation List

Patent Literature

[PTL1] JP-A-7-146233
[PTL2] JP-A-10-104160

[NPL 1] Tomohiko Yamaguchi and four others, "METHOD OF DISCRIMINATING HUE OF CONTAMINANT CONTAINED IN LUBRICANT", Fukui University, Faculty of Engineering, Research Report, March 2003, Volume 51, No. 1, pp. 81-88
[NPL 2] Tomomi Honda, "TECHNOLOGY FOR DIAGNOSING AND INSPECTING DETERIORATION OF LUBRICANT", Journal of Japan Society for Precision Engineering, 2009, Volume 75, No. 3, pp. 359-362

Summary of Invention

Technical Problem

In the technique disclosed in NPL 1 and NPL 2, it is necessary to drain lubricant from a machine and to filter the lubricant using a membrane filter. Consequently, there is a problem in that the technique lacks immediacy.

An object of the present invention is to provide a lubricant deterioration sensor which can immediately identify types and quantities of contaminant contained in lubricant of a machine.

Solution to Problem

According to an advantageous aspect of the present invention, there is provided a lubricant deterioration sensor as defined in claim 1.

The main body may have a unit replacement opening through which the replaceable unit is allowed to pass when the replaceable unit is detached from the main body. The replaceable unit may have a through-hole which causes the unit replacement opening side and an opposite side to the unit replacement opening to communicate with each other. At least a portion of the through-hole may be a screw hole.

The replaceable unit may include a detaching screw which is configured to come into contact with the main body on the opposite side to the unit replacement opening via the through-hole screwed into the screw hole from the unit replacement opening side so that the replaceable unit is separated from the main body on the opposite side to the unit replacement opening.

The replaceable unit may include a unit body which supports the light emitting element and the color light receiving element and a replaceable battery which can be replaced from the unit body. The replaceable battery may have a life span which is longer than a life span of the light emitting element, and may supply electric power to at least the light emitting element.

The replaceable unit may include a cover which covers the light emitting element and the color light receiving element. The unit body may have a battery replacement opening through which the replaceable battery is allowed to pass when the replaceable battery is detached from the unit body. The light emitting element and the color light receiving element may be arranged on an opposite side to the battery replacement opening in the cover. The replaceable battery may be arranged on the battery replacement opening side in the cover.

According to the invention, the main body includes a support member which supports the gap forming member and a fixing member which is configured to be fixed to the machine body. The first fitting portion may be disposed in the support member, and the fixing member may rotatably support the support member and the replaceable unit so that an opening direction of the oil gap varies when the support member and the replaceable unit are rotated.

A machine according to the present invention includes the above-described lubricant deterioration sensor and the machine body.

The machine may be a reducer for an industrial robot, and the machine body may be a reducer body.

The machine may be an industrial robot, and the machine body may include an arm and a reducer which is used for a joint of the arm. The lubricant may be lubricant of the reducer.

Advantageous Effects of Invention

In the lubricant deterioration sensor according to the present invention, the color light receiving element detects a color with respect to the light having a wavelength which is not absorbed by the contaminant such as ion powder contained in the lubricant in the oil gap, from the light emitted by the light emitting element. Accordingly, the color of the contaminant contained in the lubricant of the machine body can be detected immediately. That is, the lubricant deterioration sensor according to present invention can immediately identify types and quantities of the contaminant contained in the lubricant of the machine body, based on the color detected by the color light receiving element. In addition, in the lubricant deterioration sensor according to the present invention, a consumable component such as the light emitting element is provided for the replaceable unit. Accordingly, the replaceable unit is replaced from the main body, thereby enabling the life span to be lengthened.

In the lubricant deterioration sensor according to the present invention, the screw comes into contact with the main body on the opposite side to the unit replacement opening via the through-hole screwed into the screw hole of the through-hole from the unit replacement opening side so that the replaceable unit is separated from the main body on the opposite side to the unit replacement opening. Accordingly, the replaceable unit can be easily detached from the main body. Therefore, the lubricant deterioration sensor according to the present invention can facilitate replacement of the replaceable unit from the main body.

In the lubricant deterioration sensor according to the present invention, it is not necessary to separately prepare the screw which is screwed into the screw hole of the through-hole in order to detach the replaceable unit from the main body. Accordingly, the lubricant deterioration sensor can facilitate the replacement of the replaceable unit from the main body.

In the lubricant deterioration sensor according to the present invention, the replaceable battery whose life span is shorter than the life span of the light emitting element can be replaced from the unit body. Accordingly, it is possible to lengthen the life span by exchanging the replaceable battery from the unit body.

In the lubricant deterioration sensor according to the present invention, the cover hides the light emitting element and the color light receiving element from a view of a worker when the replaceable battery is replaced from the unit body. Accordingly, the lubricant deterioration sensor can facilitate the worker's replacement of the replaceable battery.

In the lubricant deterioration sensor according to the present invention, the opening direction of the oil gap when the fixing member is fixed to the machine body can be adjusted so as to improve the detection accuracy of the deterioration of the lubricant in the machine body when the fixing member is fixed to the machine body.

In the machine according to the present invention, the lubricant deterioration sensor can immediately identify the types and quantities of the contaminant contained in the lubricant in the machine body. Accordingly, it is possible to immediately predict a failure. In addition, in the machine according to the present invention, it is possible to lengthen the life span of the lubricant deterioration sensor which can immediately identify the types and quantities of the contaminant contained in the lubricant. Accordingly, it is possible to maintain accuracy in immediately predicting the failure for a longer period of time.

In the industrial robot, locus accuracy of the arm greatly depends on performance of the reducer used for the joint. Therefore, it is important to properly replace the reducer for the industrial robot when the performance becomes poor. However, when the reducer for the industrial robot is replaced, it is inevitable that the industrial robot including the reducer or an industrial robot-installed production line is to be stopped. For this reason, in order to accurately predict the time to replace the reducer for the industrial robot, it is very important that failure of the reducer for the industrial robot is predicted properly. Therefore, the reducer for the industrial robot according to the present invention provides an advantageous effect that the accuracy in immediately predicting the failure can be maintained for a longer period of time. As described above, it is very important that the failure of the reducer for the industrial robot is predicted properly. Therefore, the industrial robot according to the present invention provides an advantageous effect in that the accuracy in immediately predicting the failure can be maintained for a longer period of time.

The lubricant deterioration sensor according to the present invention can immediately identify the types and quantities of the contaminant contained in the lubricant of the machine.

Brief Description of Drawings

Fig. 1 is a side view of an industrial robot according to the present disclosure.
Fig. 2 is a cross-sectional view of a joint of the industrial robot illustrated in Fig. 1. Fig. 3 is a front view of a lubricant deterioration sensor illustrated in Fig. 2. Fig. 4 is a front sectional view of the lubricant deterioration sensor illustrated in Fig. 3 in a state where the lubricant deterioration sensor is attached to an arm. Fig. 5 is a view in which the lubricant deterioration sensor illustrated in Fig. 3 is partially broken.
Fig. 6(a) is a plan view of the lubricant deterioration sensor illustrated in Fig. 3. Fig. 6(b) is a bottom view of the lubricant deterioration sensor illustrated in Fig. 3.
Fig. 7(a) is a plan view of a housing illustrated in Fig. 3. Fig. 7(b) is a front sectional view of the housing illustrated in Fig. 3.
Fig. 8(a) is a side view of the housing illustrated in Fig. 3. Fig. 8(b) is a side sectional view of the housing illustrated in Fig. 3.
Fig. 9(a) is a plan view of the housing illustrated in Fig. 3. Fig. 9(b) is a bottom view of the housing illustrated in Fig. 3.
Fig. 10(a) is a front view of a holder illustrated in Fig. 4. Fig. 10(b) is a front sectional view of the holder illustrated in Fig. 4.
Fig. 11(a) is a side view of the holder illustrated in Fig. 4. Fig. 11(b) is a side sectional view of the holder illustrated in Fig. 4.
Fig. 12(a) is a plan view of the holder illustrated in Fig. 4. Fig. 12(b) is a bottom view of the holder illustrated in Fig. 4.
Fig. 13 is a view illustrating an optical path formed from a white LED to an RGB sensor as illustrated in Fig. 4.
Fig. 14(a) is a plan view of a housing cap illustrated in Fig. 4. Fig. 14(b) is a front sectional view of the housing cap illustrated in Fig. 4.
Fig. 15(a) is a plan view of the housing cap illustrated in Fig. 4. Fig. 15(b) is a bottom view of the housing cap illustrated in Fig. 4.
Fig. 16 is a plan view of a replaceable unit illustrated in Fig. 4 when a button-type battery and a unit cap are detached therefrom.
Fig. 17(a) is a front view of a unit body illustrated in Fig. 4. Fig. 17(b) is a front sectional view of the unit body illustrated in Fig. 4.
Fig. 18(a) is a side view of the unit body illustrated in Fig. 4. Fig. 18(b) is a side sectional view of the unit body illustrated in Fig. 4.
Fig. 19(a) is a plan view of the unit body illustrated in Fig. 4. Fig. 19(b) is a bottom view of the unit body illustrated in Fig. 4.
Fig. 20 is a cross-sectional view taken along arrow I-I in Fig. 19(a).
Fig. 21(a) is a plan view of a unit cap illustrated in Fig. 4. Fig. 21(b) is a bottom view of the unit cap illustrated in Fig. 4.
Fig. 22(a) is a front view of the unit cap illustrated in Fig. 4. Fig. 22(b) is a cross-sectional view taken along arrow II-II in Fig. 21(a).
Fig. 23 is a view illustrating a state where a button-type battery illustrated in Fig. 4 is detached therefrom.
Fig. 24 is a view illustrating a state where a replaceable unit illustrated in Fig. 4 is detached therefrom.
Fig. 25 is a view illustrating a state where a hexagon socket head cap bolt of the replaceable unit illustrated in Fig.

4 is screwed so that the replaceable unit is separated from a main body.

Fig. 26 is a view illustrating an example of a relationship between an opening direction of an oil gap illustrated in Fig. 3 with respect to lubricant flow and a color difference ΔE of a color detected by an RGB sensor with respect to the black color.

Fig. 27(a) is a view illustrating a state where the opening direction of the oil gap illustrated in Fig. 3 with respect to the lubricant flow represents 0°. Fig. 27(b) is a view illustrating a state where the opening direction of the oil gap illustrated in Fig. 3 with respect to the lubricant flow represents 45°. Fig. 27(c) is a view illustrating a state where the opening direction of the oil gap illustrated in Fig. 3 with respect to the lubricant flow represents 90°.

Description of Embodiments

Hereinafter, an embodiment of the present invention will be described with reference to the drawings.

First, a configuration of an industrial robot serving as a machine according to the present disclosure will be described.

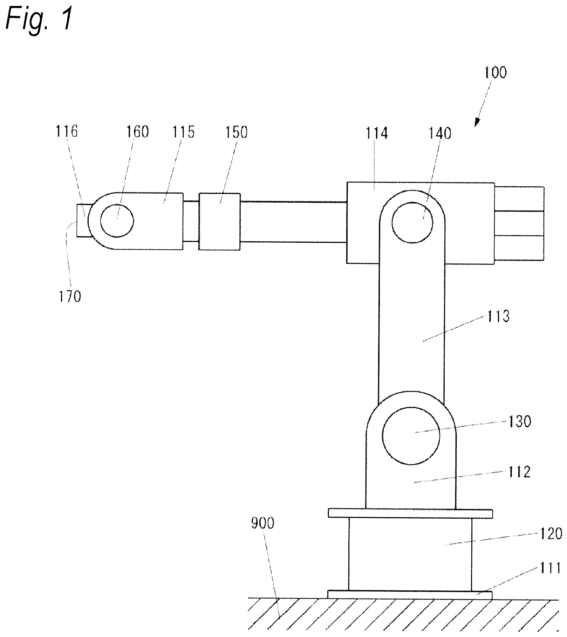

Fig. 1 is a side view of an industrial robot 100 according to the present disclosure.

As illustrated in Fig. 1, the industrial robot 100 includes an attachment section 111 attached to an installation section 900 such as a floor and a ceiling, arms 112 to 116, a joint 120 connecting the attachment section 111 and the arm 112, a joint 130 connecting the arm 112 and the arm 113, a joint 140 connecting the arm 113 and the arm 114, a joint 150 connecting the arm 114 and the arm 115, a joint 160 connecting the arm 115 and the arm 116, and a joint 170 connecting the arm 116 and a hand (not illustrated).

A part in which lubricant such as lubricant 131a (to be described later) and a lubricant deterioration sensor such as lubricant deterioration sensors 137a, 137b, 139a, and 139b (to be described later) are excluded from the industrial robot 100 configures a machine body according to the present invention when the industrial robot 100 is a machine according to the present invention.

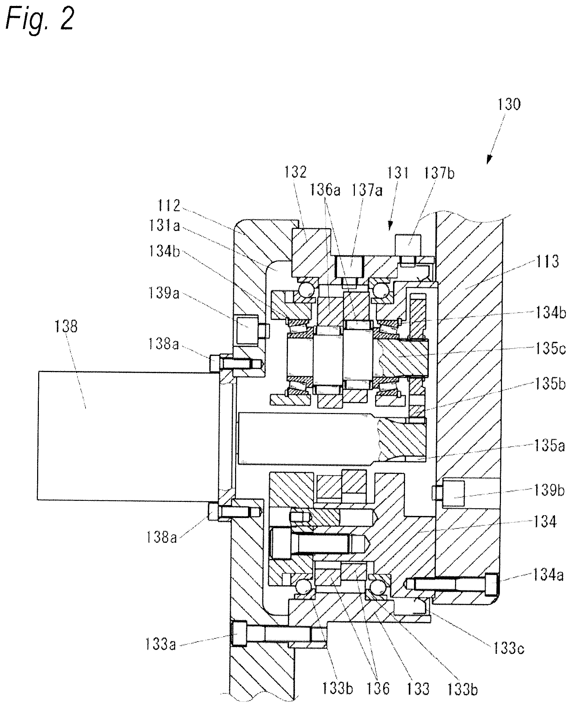

Fig. 2 is a cross-sectional view of the joint 130. Hereinafter, the joint 130 will be described, but the joints 120 and 140 to 170 have the same configuration.

As illustrated in Fig. 2, the joint 130 includes a reducer 131 which is a reducer for the industrial robot connecting the arm 112 and the arm 113, a motor 138 fixed to the arm 112 by a bolt 138a, and the lubricant deterioration sensor 139a and the lubricant deterioration sensor 139b which are used for detecting deterioration of lubricant 131a which decreases friction generated in a movable portion of the reducer 131.

The reducer 131 includes a reducer body 132, and the lubricant deterioration sensor 137a and the lubricant deterioration sensor 137b which are used for detecting the deterioration of the lubricant 131a in the reducer body 132. The reducer body 132 configures a machine body according to the present invention when the reducer 131 is the machine according to the present invention.

The reducer body 132 includes a case 133 fixed to the arm 112 by a bolt 133a, a support body 134 fixed to the arm 113 by a bolt 134a, a gear 135a fixed to an output shaft of the motor 138, three gears 135b (only one is illustrated in Fig. 2) which are arranged around the central axis of the reducer 131 at equal intervals and mesh with the gear 135a, three crank shafts 135c (only one is illustrated in Fig. 2) which are arranged around the central axis of the reducer 131 at equal intervals and are fixed to the gears 135b, and two external gears 136 which mesh with an internal gear disposed in the case 133.

The support body 134 is rotatably supported by the case 133 via a bearing 133b. A seal member 133c for preventing leakage of the lubricant 131a is disposed between the case 133 and the support body 134.

The crank shaft 135c is rotatably supported by the support body 134 via a bearing 134b, and is rotatably supported by the external gear 136 via a bearing 136a.

The lubricant deterioration sensor 137a and the lubricant deterioration sensor 137b are fixed to the case 133. The lubricant deterioration sensor 139a is fixed to the arm 112. The lubricant deterioration sensor 139b is fixed to the arm 113.

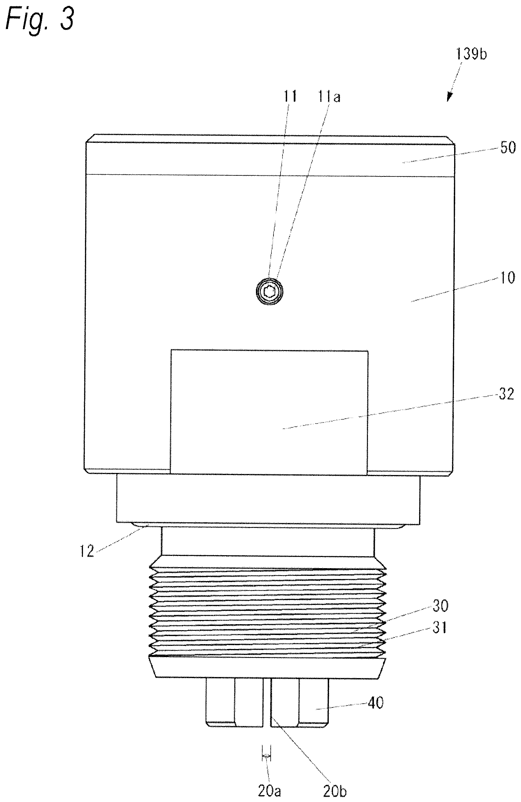

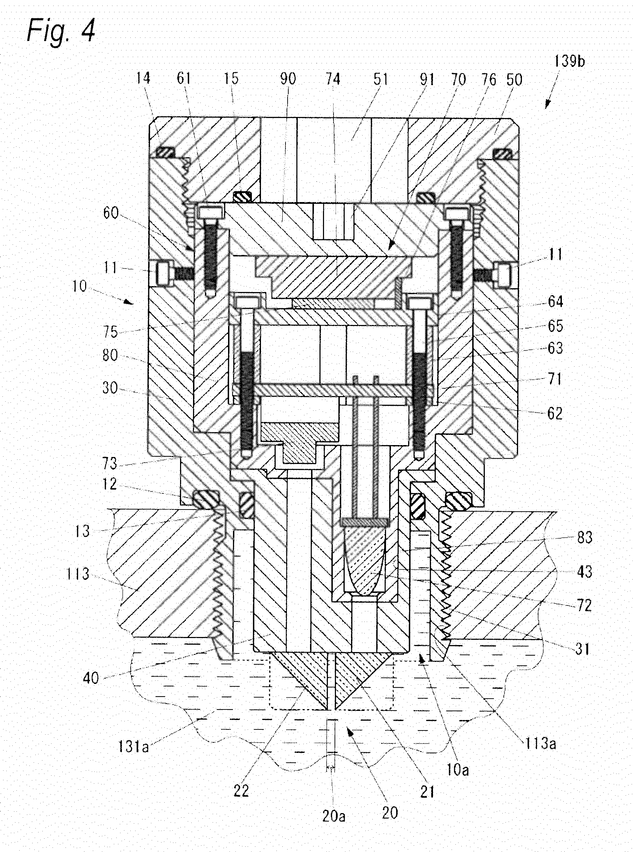

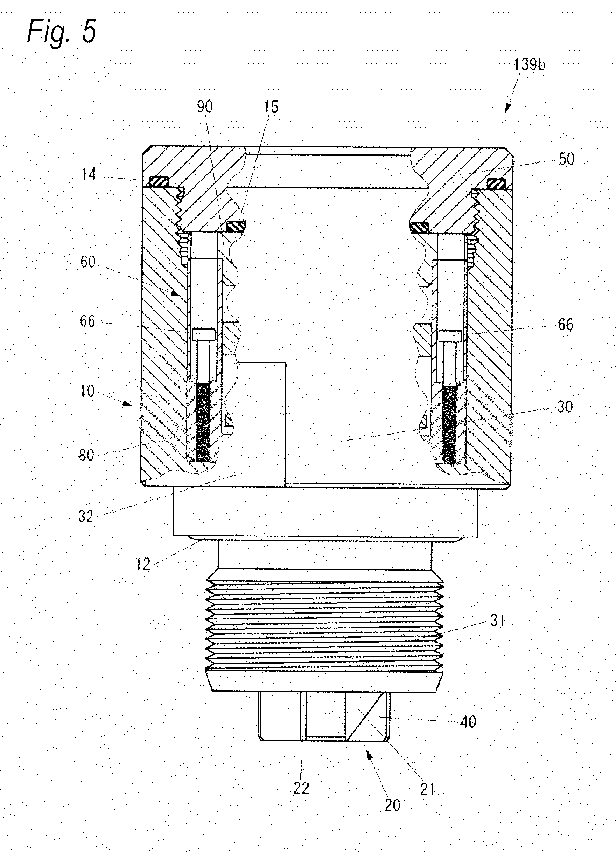

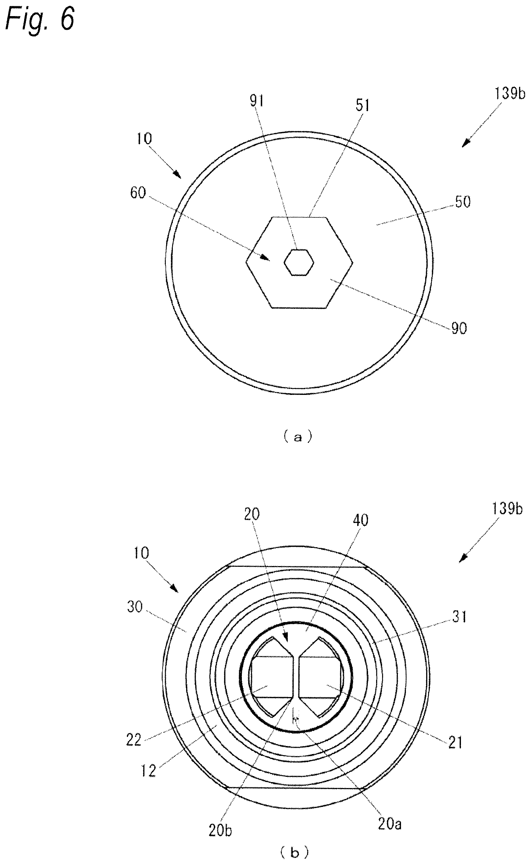

Fig. 3 is a front view of the lubricant deterioration sensor 139b. Fig. 4 is a front sectional view of the lubricant deterioration sensor 139b in a state where the lubricant deterioration sensor 139b is attached to the arm 113. Fig. 5 is a view in which the lubricant deterioration sensor 139b is partially broken. Fig. 6(a) is a plan view of the lubricant deterioration sensor 139b. Fig. 6(b) is a bottom view of the lubricant deterioration sensor 139b. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

As illustrated in Figs. 3 to 6(b), the lubricant deterioration sensor 139b includes the arm 113 of the industrial robot 100, that is, the main body 10 fixed to the machine body, and a replaceable unit 60 which can be replaced from the main body 10.

The main body 10 includes a gap forming member 20 having an oil gap 20a serving as a gap which the lubricant 131a is allowed to enter, an aluminum alloy-made housing 30 serving as a fixing member to be fixed to the arm 113, an aluminum alloy-made holder 40 serving as a support member for supporting the gap forming member 20, and an aluminum alloy-made housing cap 50 which accommodates the replaceable unit 60 with the housing 30. The main body 10 includes a hexagon socket head cap bolt 11 which prevents rotation of the replaceable unit 60 with respect to the housing 30 by coming into contact with both the housing 30 and the replaceable unit 60, an O-ring 12 which prevents the lubricant 131a from leaking out from a portion between the housing 30 and the arm 113, an O-ring 13 which is arranged between the housing 30 and the holder 40, an O-ring 14 which is arranged between the housing 30 and the housing cap 50, and an O-ring 15 which is arranged between the housing cap 50 and the replaceable unit 60.

The hexagon socket head cap bolt 11 includes a jig contact portion 11a which comes into contact with a jig such as a hexagon wrench. The jig contact portion 11a is a portion in which a drive force for coming into contact with both the housing 30 and the replaceable unit 60 is received from the outside by using a contact force. The jig contact portion 11a is arranged at a position where the lubricant 131a is not touched when the housing 30 is fixed to the arm 113.

The replaceable unit 60 includes an electronic component group 70, an aluminum alloy-made unit body 80 which supports the electronic component group 70, and an aluminum alloy-made unit cap 90 which accommodates the electronic component group 70 with the unit body 80. The replaceable unit 60 includes a hexagon socket head cap bolt 61 which fixes the unit body 80 and the unit cap 90, a spacer 62 which is arranged between a circuit board 71 (to be described later) and the unit body 80, a spacer 63 which is arranged on an opposite side to the spacer 62 in the circuit board 71, an aluminum alloy-made cover 64 which is arranged on an opposite side to the circuit board 71 in the spacer 63, a hexagon socket head cap bolt 65 which fixes the spacer 62, the circuit board 71, the spacer 63, and the cover 64 to the unit body 80, and a hexagon socket head cap bolt 66 serving as a detaching screw for detaching the replaceable unit 60 from the main body 10.

The gap forming member 20 is configured to have two glass-made right-angle prisms 21 and 22. The oil gap 20a is formed between the two right-angle prisms 21 and 22.

The electronic component group 70 includes the circuit board 71, a white LED 72 mounted on the circuit board 71, an RGB sensor 73 mounted on the circuit board 71, a button-type battery 74 which supplies electric power to each electronic component on the circuit board 71, such as the white LED 72 and the RGB sensor 73, and terminals 75 and 76 for electrically connecting the button-type battery 74 and the circuit board 71 to each other. In addition to the white LED 72 and the RGB sensor 73, multiple electronic components such as a communication device which performs communication with an external device of the lubricant deterioration sensor 139b are mounted on the circuit board 71.

The button-type battery 74 can be replaced from the unit body 80, and configures a replaceable battery according to the present invention. The button-type battery 74 has a life span shorter than a life span of the white LED 72.

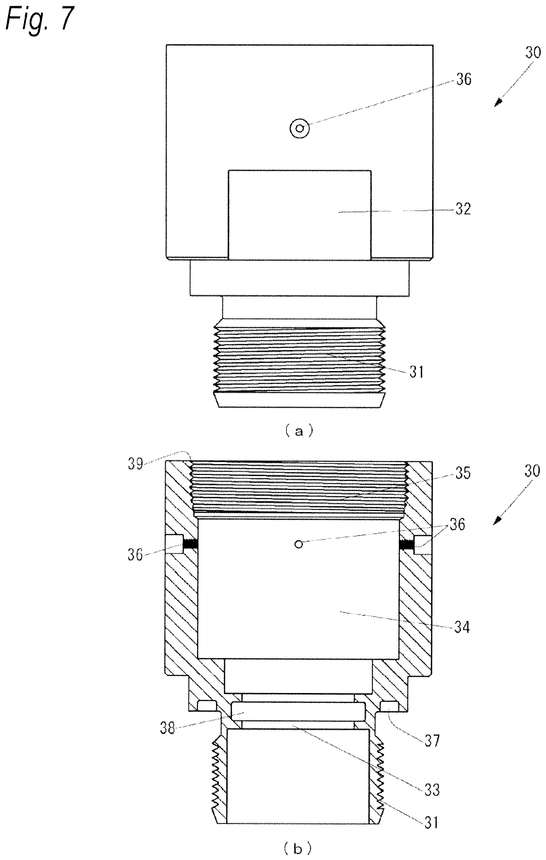

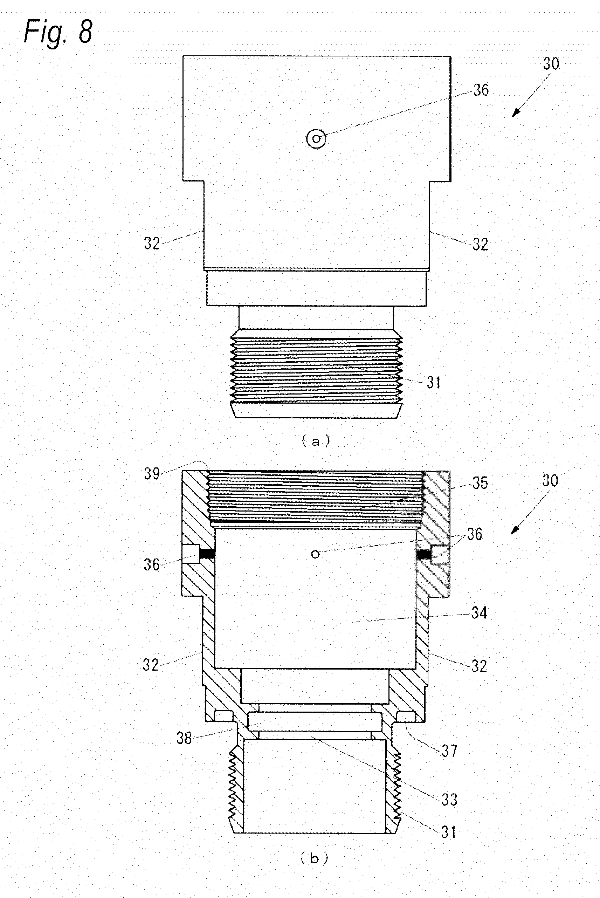

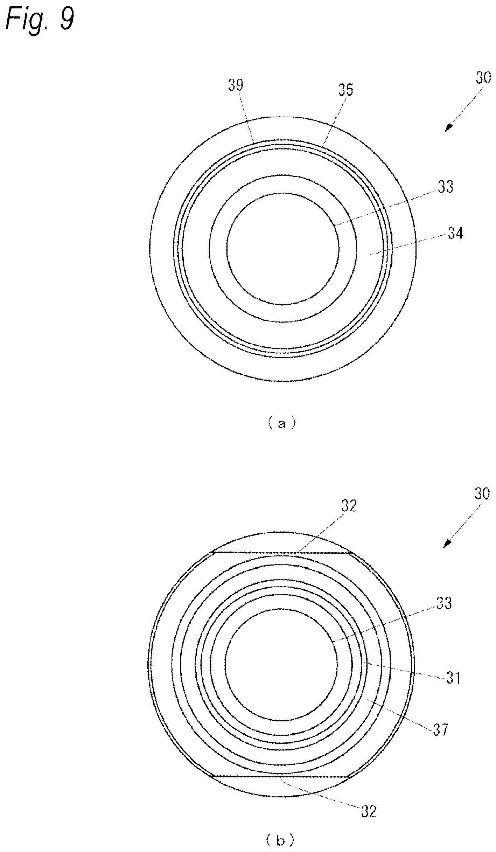

Fig. 7(a) is a front view of the housing 30. Fig. 7(b) is a front sectional view of the housing 30. Fig. 8(a) is a side view of the housing 30. Fig. 8(b) is a side sectional view of the housing 30. Fig. 9(a) is a plan view of the housing 30. Fig. 9(b) is a bottom view of the housing 30.

As illustrated in Figs. 3 to 9(b), the housing 30 includes a screw 31 fixed to a screw hole 113a of the arm 113, a jig contact portion 32 to be gripped by a jig such as a spanner when the screw 31 is rotated with respect to the screw hole 113a of the arm 113, a holder accommodation portion 33 which accommodates the holder 40, and a unit accommodation portion 34 which accommodates the replaceable unit 60. In addition, the housing 30 has a female screw 35 into which the housing cap 50 is screwed, a screw hole 36 into which the hexagon socket head cap bolt 11 is screwed, a groove 37 into which the O-ring 12 is fitted, a groove 38 into which the O-ring 13 is fitted, and a unit replacement opening 39 through which the replaceable unit 60 is allowed to pass when the replaceable unit 60 is detached.

When the lubricant deterioration sensor 139b is detached therefrom, the screw hole 113a of the arm 113 may be used in supplying the lubricant 131a to the reducer 131 and in discarding the lubricant 131a from the reducer 131.

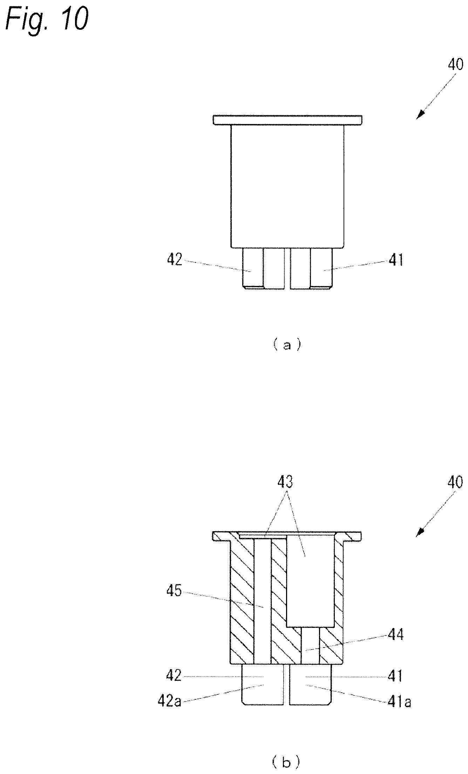

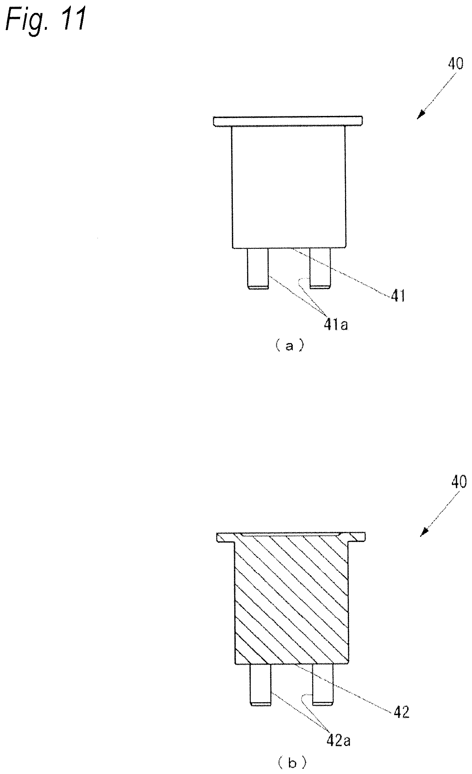

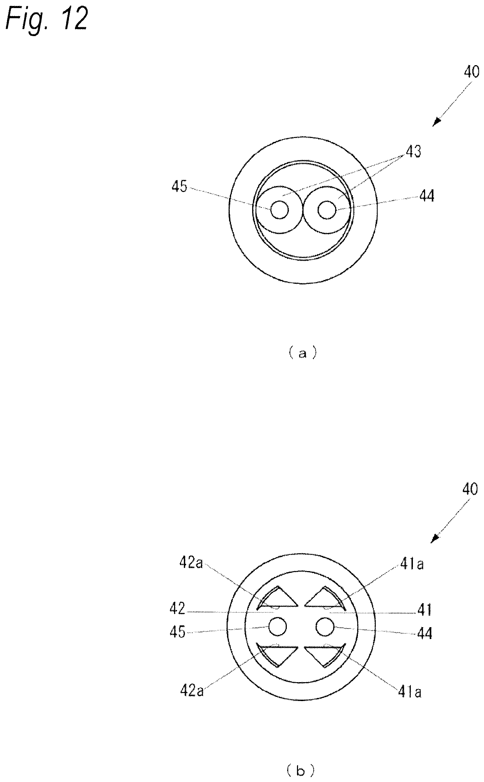

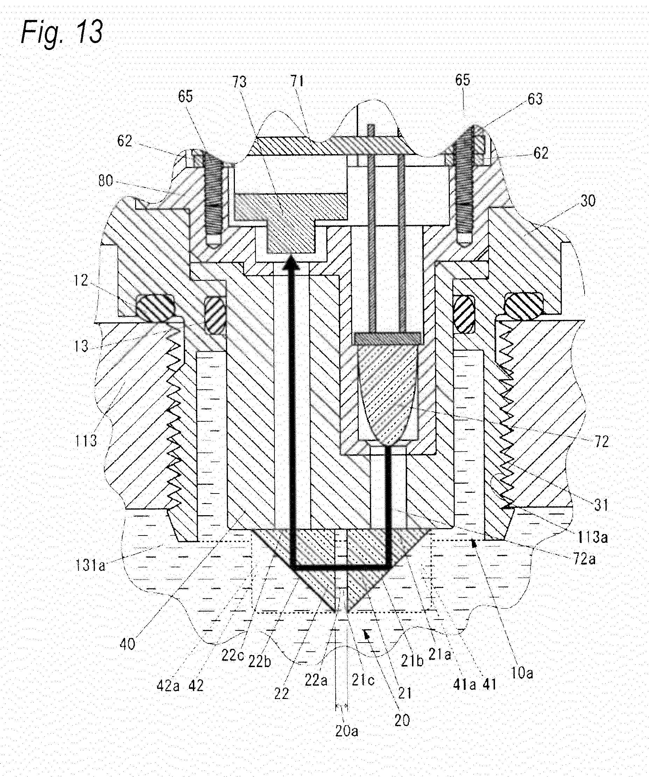

Fig. 10(a) is a front view of the holder 40. Fig. 10(b) is a front sectional view of the holder 40. Fig. 11(a) is a side view of the holder 40. Fig. 11(b) is a side sectional view of the holder 40. Fig. 12(a) is a plan view of the holder 40. Fig. 12(b) is a bottom view of the holder 40. Fig. 13 is a view illustrating an optical path 72a formed from the white LED 72 to the RGB sensor 73.

As illustrated in Figs. 6(b) and 10(a) to 13, the holder 40 includes a prism accommodation portion 41 which accommodates the right-angle prism 21, a prism accommodation portion 42 which accommodates the right-angle prism 22, and a fitting portion 43 (first fitting portion) which is fitted to the unit body 80 in a state where a positional relationship of the gap forming member 20 with the white LED 72 and the RGB sensor 73 becomes a predetermined positional relationship. In addition, the holder 40 has a hole 44 which causes the prism accommodation portion 41 and the fitting portion 43 to communicate with each other, and a hole 45 which causes the prism accommodation portion 42 and the fitting portion 43 to communicate with each other.

The holder 40 is arranged inside the screw 31 so as to be separate from the screw 31 so that a portion of the optical path 72a is arranged inside the screw 31 of the housing 30. That is, a gap 10a is formed between the screw 31 of the housing 30 and the holder 40.

The prism accommodation portion 41 includes two walls 41a which interpose the right-angle prism 21 therebetween, and fixes the right-angle prism 21 on the walls 41a by using an adhesive. The prism accommodation portion 42 includes two walls 42a which interpose the right-angle prism 22 therebetween, and fixes the right-angle prism 22 on the walls 42a by using an adhesive.

The holder 40 surrounds at least a portion of the optical path 72a formed from the white LED 72 to the RGB sensor 73 by using the hole 44, the prism accommodation portion 41, the prism accommodation portion 42, and the hole 45. For example, a surface of the holder 40 is subjected to treatment for preventing light reflection such as matt-finishing black alumite treatment.

As illustrated in Fig. 13, the oil gap 20a of the gap forming member 20 is arranged on the optical path 72a formed from the white LED 72 to the RGB sensor 73.

The right-angle prisms 21 and 22 allow light emitted by the white LED 72 to pass therethrough. The right-angle prism 21 has a light incident surface 21a on which the light emitted by the white LED 72 is incident, a light reflection surface 21b which reflects the light incident from the light incident surface 21a and bends the light travelling direction by 90 degrees, and a light emitting surface 21c from which the light reflected on the light reflection surface 21b is emitted. The right-angle prism 22 has a light incident surface 22a on which the light emitted from the light emitting surface 21c of the right-angle prism 21 is incident, a light reflection surface 22b which reflects the light incident from the light incident surface 22a and bends the light travelling direction by 90 degrees, and a light emitting surface 22c from which the light reflected on the light reflection surface 22b is emitted.

The light incident surface 21a, the light reflection surface 21b, the light emitting surface 21c of the right-angle prism 21, and the light incident surface 22a, the light reflection surface 22b, and the light emitting surface 22c of the right-angle prism 22 are subjected to optical polishing. In addition, an aluminum vapor deposition film is formed on the light reflection surface 21b of the right-angle prism 21 and the light reflection surface 22b of the right-angle prism 22. Then, in order to protect the aluminum vapor deposition film whose rigidity and adhesion force are weak, a SiO2 film is further formed on the aluminum vapor deposition film.

The optical path 72a is bent by 90 degrees on the light reflection surface 21b of the right-angle prism 21 and is also bent by 90 degrees on the light reflection surface 22b of the right-angle prism 22. That is, the optical path 72a is bent by 180 degrees by the gap forming member 20.

In other words, a distance between the light emitting surface 21c of the right-angle prism 21 and the light incident surface 22a of the right-angle prism 22 is a length of the oil gap 20a. If the length of the oil gap 20a is too short, the contaminant contained in the lubricant 131a is less likely to properly flow through the oil gap 20a. Consequently, accuracy in detecting a color of the contaminant contained in the lubricant 131a becomes poor. In contrast, if the length of the oil gap 20a is too long, the light emitted from the white LED 72 is excessively absorbed by the contaminant contained in the lubricant 131a inside the oil gap 20a, and is less likely to be transferred to the RGB sensor 73. Consequently, the accuracy in detecting a color of the contaminant contained in the lubricant 131a also becomes poor. Therefore, it is preferable to properly set the length of the oil gap 20a so as to improve the accuracy in detecting a color of the contaminant contained in the lubricant 131a. For example, the length of the oil gap 20a is 1 mm.

The white LED 72 is an electronic component which emits white light, and configures a light emitting element according to the present invention. For example, as the white LED 72, a product type of NSPW500GS-K1 manufactured by Nichia Corporation may be used.

The RGB sensor 73 is an electronic component which detects a color of the received light, and configures a color light receiving element according to the present invention. For example, as the RGB sensor 73, a product type of S9032-02 manufactured by Hamamatsu Photonics K.K. may be used.

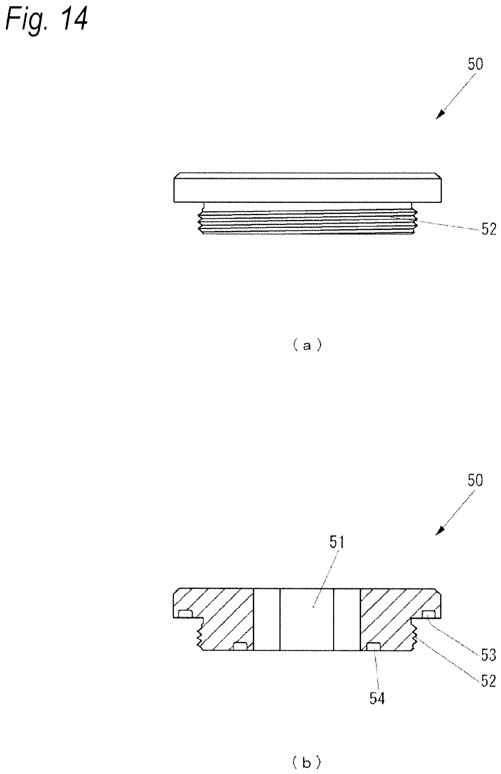

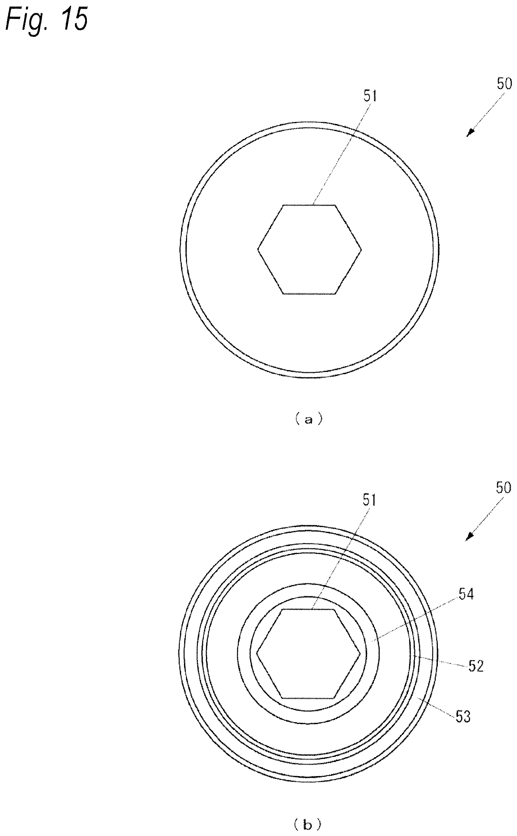

Fig. 14(a) is a plan view of the housing cap 50. Fig. 14(b) is a front sectional view of the housing cap 50. Fig. 15(a) is a plan view of the housing cap 50. Fig. 15(b) is a bottom view of the housing cap 50.

As illustrated in Figs. 14(a) to 15(b), the housing cap 50 includes a jig contact portion 51 which comes into contact with a jig such as a hexagon wrench when the housing cap 50 is attached to the housing 30. The jig contact portion 51 is a portion in which a drive force of the rotation of the housing cap 50 with respect to the housing 30 is received from the outside by using a contact force. The jig contact portion 51 is arranged at a position where the lubricant 131a is not touched when the housing 30 is fixed to the arm 113. In addition, the housing cap 50 has a male screw 52 to be screwed into the female screw 35 of the housing 30, a groove 53 to which the O-ring 14 is fitted, and a groove 54 to which the O-ring 15 is fitted.

For example, a surface of the housing cap 50 is subjected to treatment for preventing light reflection such as matt-finishing black alumite treatment.

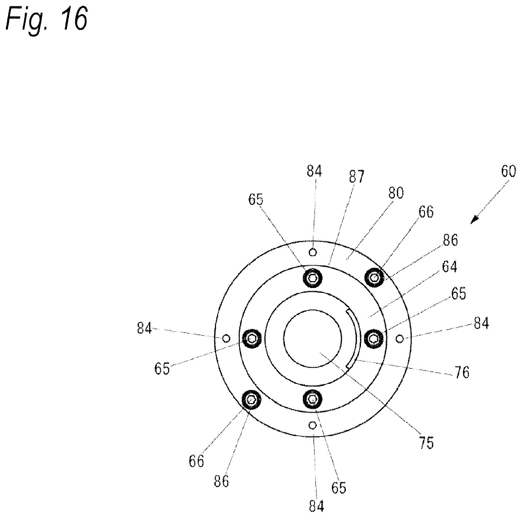

Fig. 16 is a plan view of the replaceable unit 60 when the button-type battery 74 and the unit cap 90 are detached therefrom.

As illustrated in Figs. 4 and 16, the white LED 72 and the RGB sensor 73 are arranged on an opposite side to a battery replacement opening 87 (to be described later) in the cover 64 of the replaceable unit 60. In addition, the button-type battery 74 is arranged on the battery replacement opening 87 side in the cover 64. That is, the cover 64 is arranged between the white LED 72 and the RGB sensor 73, and the button-type battery 74, and covers the white LED 72 and the RGB sensor 73 from the battery replacement opening 87 side.

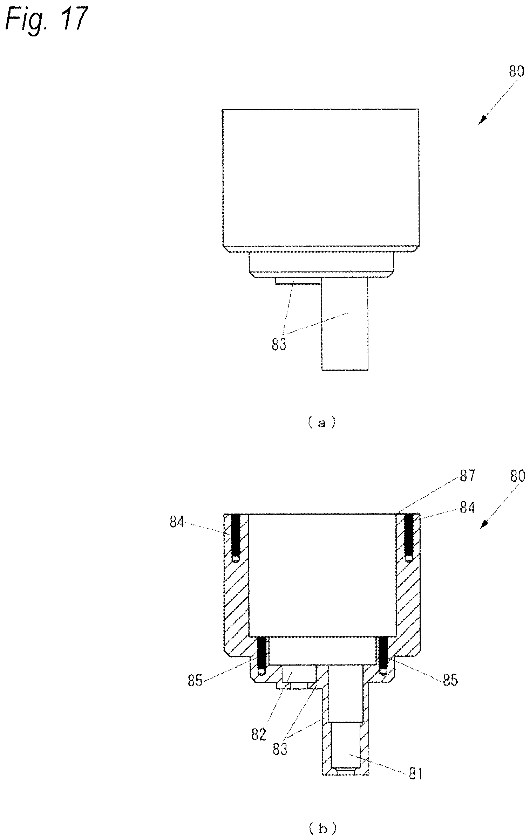

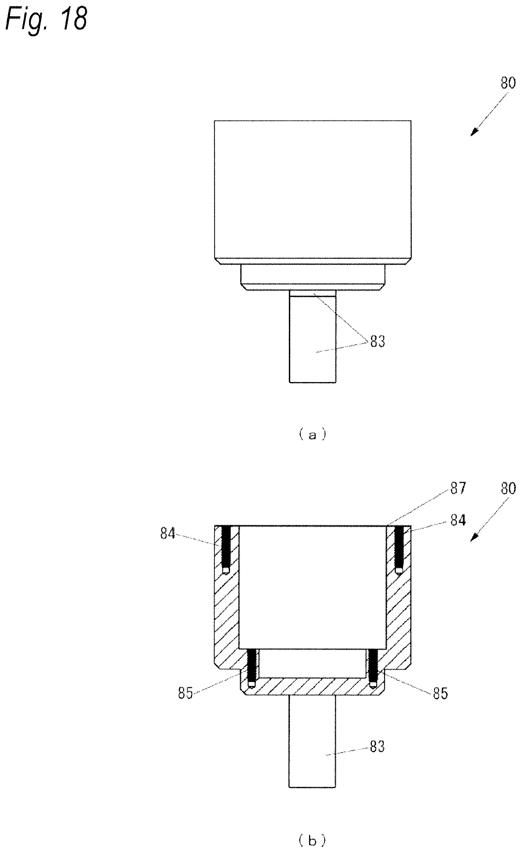

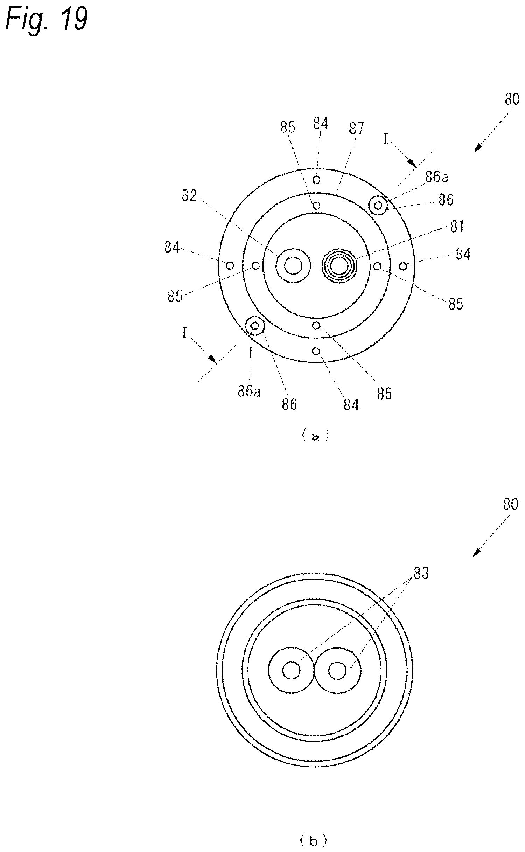

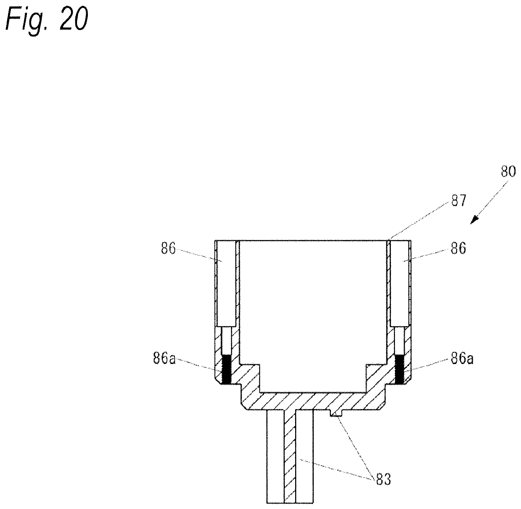

Fig. 17(a) is a front view of the unit body 80. Fig. 17(b) is a front sectional view of the unit body 80. Fig. 18(a) is a side view of the unit body 80. Fig. 18(b) is a side sectional view of the unit body 80. Fig. 19(a) is a plan view of the unit body 80. Fig. 19(b) is a bottom view of the unit body 80. Fig. 20 is a cross-sectional view taken along arrow I-I in Fig. 19(a).

As illustrated in Figs. 4 and 17(a) to 20, the unit body 80 includes an LED accommodation portion 81 which accommodates the white LED 72, an RGB sensor accommodation portion 82 which accommodates the RGB sensor 73, and a fitting portion 83 (second fitting portion) which is fitted to the fitting portion 43 of the holder 40 in a state where a positional relationship of the gap forming member 20 with the white LED 72 and the RGB sensor 73 becomes a predetermined positional relationship. In addition, the unit body 80 has a screw hole 84 into which the hexagon socket head cap bolt 61 is screwed, a screw hole 85 into which the hexagon socket head cap bolt 65 is screwed, a through-hole 86 which causes the unit replacement opening 39 side of the housing 30 when accommodated in the housing 30 to communicate with an opposite side to the unit replacement opening 39, and a battery replacement opening 87 through which the button-type battery 74 is allowed to pass when the button-type battery 74 is detached. A portion of the through-hole 86 serves as a screw hole 86a into which the hexagon socket head cap bolt 66 is screwed.

The unit body 80 supports the circuit board 71 via the spacer 62, and supports the white LED 72 and the RGB sensor 73 via the circuit board 71.

For example, a surface of the unit body 80 is subjected to treatment for preventing light reflection such as matt-finishing black alumite treatment.

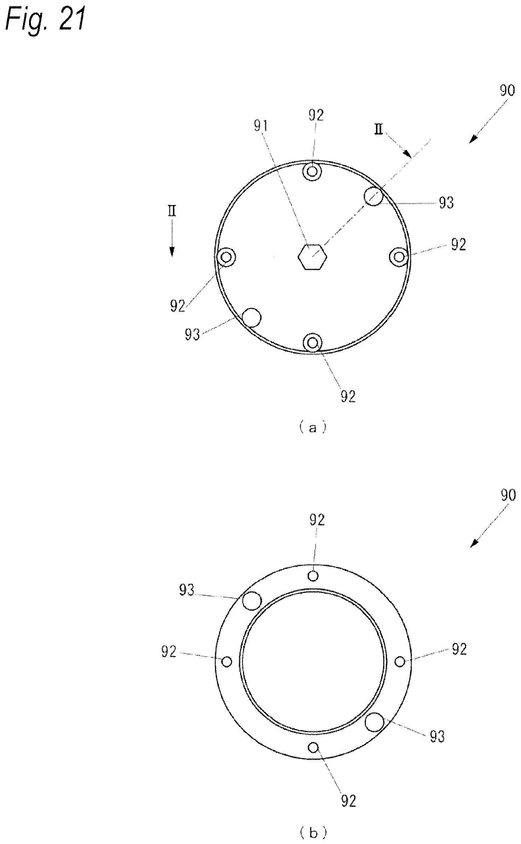

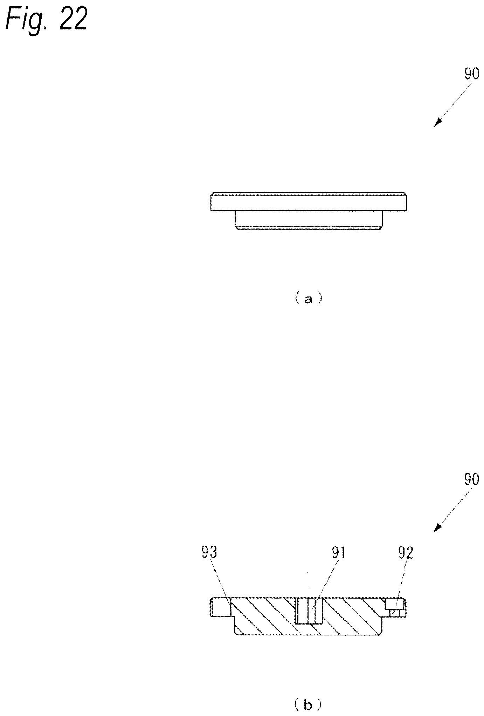

Fig. 21(a) is a plan view of the unit cap 90. Fig. 21(b) is a bottom view of the unit cap 90. Fig. 22(a) is a front view of the unit cap 90. Fig. 22(b) is a cross-sectional view taken along arrow II-II in Fig. 21(a).

As illustrated in Figs. 21(a) to 22(b), the unit cap 90 includes a jig contact portion 91 which comes into contact with a jig such as a hexagon wrench when the replaceable unit 60 is rotated with respect to the housing 30. The jig contact portion 91 is a portion in which a drive force of the rotation of the replaceable unit 60 with respect to the housing 30 is received from the outside by using a contact force. The jig contact portion 91 is arranged at a position where the lubricant 131a is not touched when the housing 30 is fixed to the arm 113. In addition, the unit cap 90 has a hole 92 into which the hexagon socket head cap bolt 61 is inserted, and a hole 93 which communicates with the through-hole 86 of the unit body 80.

For example, a surface of the unit cap 90 is subjected to treatment for preventing light reflection such as matt-finishing black alumite treatment.

Next, an assembly method of the lubricant deterioration sensor 139b will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

First, an adhesive adheres to a surface which comes into contact with the light incident surface 21a of the right-angle prism 21, within the prism accommodation portion 41 of the holder 40, and two surfaces which respectively come into contact with the two walls 41a of the prism accommodation portion 41, out of the surfaces of the right-angle prism 21. Then, the adhesive fixes the right-angle prism 21 to the prism accommodation portion 41. In addition, an adhesive adheres to a surface which comes into contact with the light emitting surface 22c of the right-angle prism 22, within the prism accommodation portion 42 of the holder 40, and two surfaces which respectively come into contact with the two walls 42a of the prism accommodation portion 42, out of the surfaces of the right-angle prism 22. Then, the adhesive fixes the right-angle prism 22 to the prism accommodation portion 42. In the above-described manner, the holder 40 having the gap forming member 20 attached thereto is assembled.

In addition, the white LED 72 is fixed to the LED accommodation portion 81 of the unit body 80 by using an adhesive. Then, the circuit board 71 on which the RGB sensor 73 is mounted is temporarily fixed to the unit body 80 via the spacer 62 by using a hexagon socket head cap bolt (not illustrated), and the white LED 72 is fixed to the circuit board 71 by means of soldering. The hexagon socket head cap bolt is screwed into the screw hole 85 of the unit body 80 in place of the hexagon socket head cap bolt 65, and has a length shorter than a length of the hexagon socket head cap bolt 65 in order to temporarily fix the spacer 62 and the circuit board 71 to the unit body 80. After the white LED 72 is fixed to the circuit board 71 by means of soldering, the hexagon socket head cap bolt is detached, and the circuit board 71 is fixed to the unit body 80 via the spacer 62, the spacer 63, and the cover 64 by using the hexagon socket head cap bolt 65. At this time, the terminals 75 and 76 which are arranged in the cover 64, and the circuit board 71 are electrically connected to each other by a wire (not illustrated). Then, in a state where the button-type battery 74 is electrically connected to the terminals 75 and 76 which are arranged in the cover 64, the unit cap 90 is fixed to the unit body 80 by using the hexagon socket head cap bolt 61. In addition, the hexagon socket head cap bolt 66 is screwed into the screw hole 86a of the unit body 80 in a state where the hexagon socket head cap bolt 66 does not protrude from the through-hole 86. In the above-described manner, the replaceable unit 60 having the hexagon socket head cap bolt 66 and the electronic component group 70 attached thereto is assembled.

Thereafter, the holder 40 is accommodated in the holder accommodation portion 33 of the housing 30 to which the O-ring 12 and the O-ring 13 are attached.

Then, the replaceable unit 60 is accommodated in the unit accommodation portion 34 of the housing 30. At this time, the fitting portion 43 of the holder 40 of the main body 10 and the fitting portion 83 of the unit body 80 of the replaceable unit 60 are fitted to each other. In this manner, a positional relationship of the gap forming member 20 of the main body 10 with the white LED 72 and the RGB sensor 73 of the replaceable unit 60 becomes a predetermined positional relationship.

Then, the housing cap 50 to which the O-ring 14 and the O-ring 15 are attached is rotated with respect to the housing 30 by using a jig which is inserted into the jig contact portion 51. In this manner, the male screw 52 of the housing cap 50 is screwed into the female screw 35 of the housing 30, thereby fixing the housing cap 50 to the housing 30.

Lastly, the replaceable unit 60 is fixed to the main body 10 by using the hexagon socket head cap bolt 11.

Next, a method of installing the lubricant deterioration sensor 139b in the arm 113 will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

The jig contact portion 32 of the housing 30 is gripped by a jig, and the screw 31 of the housing 30 is screwed into the screw hole 113a of the arm 113, thereby fixing the lubricant deterioration sensor 139b to the arm 113.

Next, a method of replacing the button-type battery 74 of the lubricant deterioration sensor 139b will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

First, the housing cap 50 is rotated with respect to the housing 30 by using a jig which is inserted into the jig contact portion 51, thereby detaching the housing cap 50 from the housing 30.

Then, the hexagon socket head cap bolt 61 is detached, thereby detaching the unit cap 90 from the unit body 80.

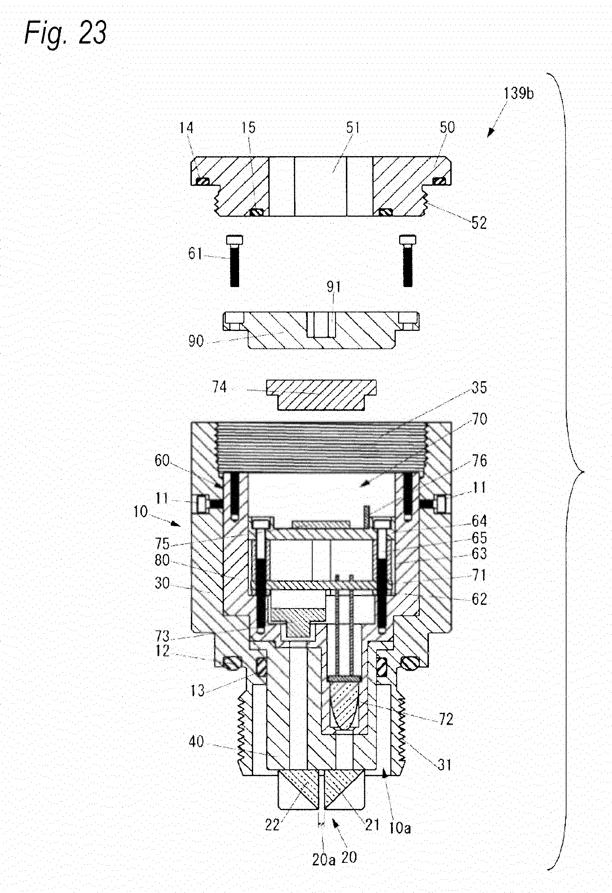

Then, as illustrated in Fig. 23, the button-type battery 74 is separated from the terminals 75 and 76 arranged in the cover 64, thereby detaching the button-type battery 74 from the unit body 80.

Then, a new button-type battery 74 is electrically connected to the terminals 75 and 76 arranged in the cover 64.

Then, the unit cap 90 is fixed to the unit body 80 by using the hexagon socket head cap bolt 61.

Lastly, the housing cap 50 is rotated with respect to the housing 30 by using a jig which is inserted into the jig contact portion 51. In this manner, the male screw 52 of the housing cap 50 is screwed into the female screw 35 of the housing 30, thereby fixing the housing cap 50 to the housing 30.

Next, a method of replacing the replaceable unit 60 of the lubricant deterioration sensor 139b will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

First, the housing cap 50 is rotated with respect to the housing 30 by using a jig which is inserted into the jig contact portion 51, thereby detaching the housing cap 50 from the housing 30.

Then, the hexagon socket head cap bolt 11 is unfastened by using a jig which is inserted into the jig contact portion 11a.

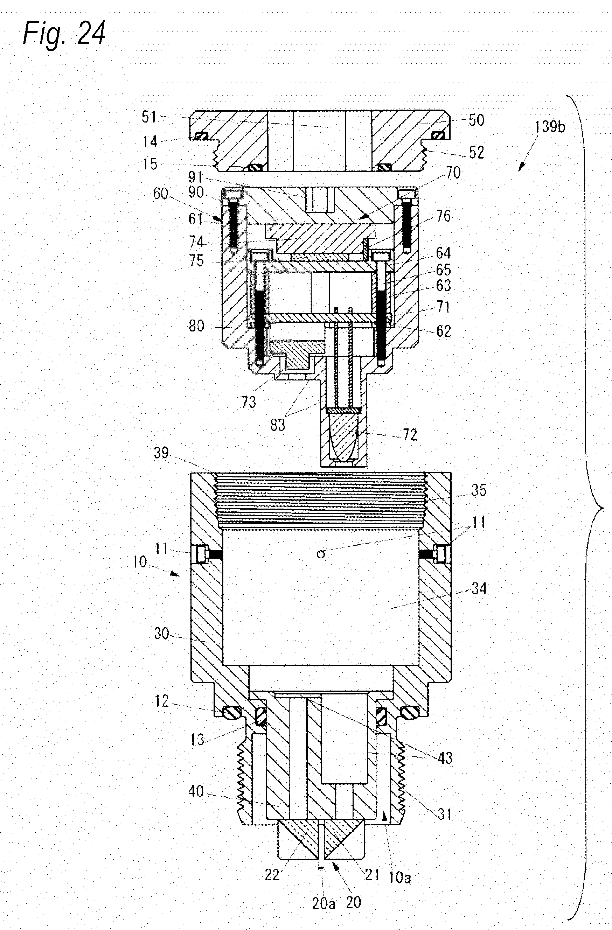

Then, as illustrated in Fig. 24, the replaceable unit 60 is detached from the unit accommodation portion 34 of the housing 30 via the unit replacement opening 39 of the housing 30.

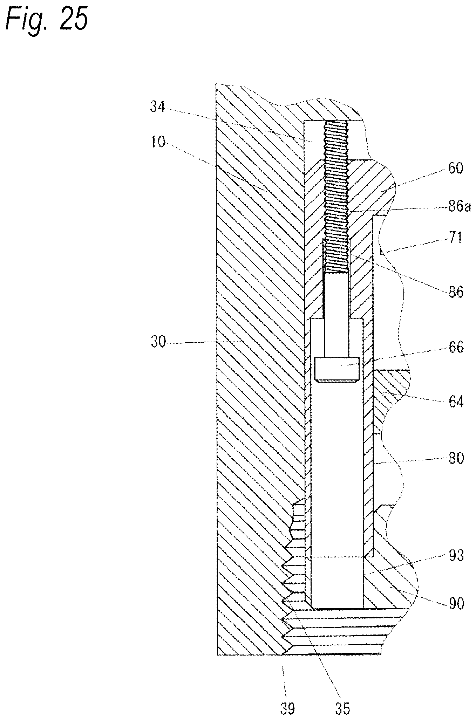

Here, if the housing 30 is fixed so that the unit replacement opening 39 is positioned below in a vertical direction, the replaceable unit 60 falls down from the unit accommodation portion 34 of the housing 30 by means of its own weight of the replaceable unit 60. However, if for some reason, the replaceable unit 60 does not fall down from the unit accommodation portion 34 of the housing 30 by means of its own weight of the replaceable unit 60, a worker causes the hexagon socket head cap bolt 66 of the replaceable unit 60 to be screwed into the screw hole 86a of the through-hole 86 of the replaceable unit 60, thereby enabling the replaceable unit 60 to be detached from the main body 10. That is, as illustrated in Fig. 25, the hexagon socket head cap bolt 66 of the replaceable unit 60 is screwed into the screw hole 86a of the through-hole 86 of the replaceable unit 60, and comes into contact with the main body 10 on the opposite side to the unit replacement opening 39 side via the through-hole 86. In this manner, the replaceable unit 60 is separated from the main body 10 on the opposite side to the unit replacement opening 39 side. This configuration enables the replaceable unit 60 to be reliably detached from the main body 10 regardless of whether or not the housing 30 is fixed so that the unit replacement opening 39 is located below in the vertical direction. Therefore, for example, the replaceable unit 60 can be reliably detached from the main body 10 while the housing 30 is attached to the arm 113 as it is.

Then, a new replaceable unit 60 is accommodated in the unit accommodation portion 34 of the housing 30 via the unit replacement opening 39 of the housing 30. At this time, the fitting portion 43 of the holder 40 of the main body 10 and the fitting portion 83 of the unit body 80 of the replaceable unit 60 are fitted to each other. In this manner, a positional relationship of the gap forming member 20 of the main body 10 with the white LED 72 and the RGB sensor 73 of the replaceable unit 60 becomes a predetermined positional relationship.

Then, the housing cap 50 is rotated with respect to the housing 30 by using a jig which is inserted into the jig contact portion 51. In this manner, the male screw 52 of the housing cap 50 is screwed into the female screw 35 of the housing 30, thereby fixing the housing cap 50 to the housing 30.

Lastly, the new replaceable unit 60 is fixed to the main body 10 by using the hexagon socket head cap bolt 11.

Next, an operation of the industrial robot 100 will be described.

First, an operation of the joint 130 will be described. Hereinafter, the joint 130 will be described, but the joints 120 and 140 to 170 have the same configuration.

If the output shaft of the motor 138 in the joint 130 is rotated, a rotation force of the motor 138 is reduced by the reducer 131, thereby moving the arm 113 fixed to the support body 134 of the reducer 131 to the arm 112 fixed to the case 133 of the reducer 131.

Next, an operation of the lubricant deterioration sensor 139b will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

The lubricant deterioration sensor 139b causes the white LED 72 to emit white light by using electric power supplied from the button-type battery 74.

Then, the lubricant deterioration sensor 139b outputs luminous energy of each RGB color of the light received by the RGB sensor 73 to an external device as an electric signal, by radio using a communication device on the circuit board 71.

The lubricant deterioration sensor 139b may include a separately mounted sensor in addition to the RGB sensor 73. For example, when a temperature sensor for detecting a temperature of the lubricant 131a is included in the electronic component group 70, the lubricant deterioration sensor 139b can also output the temperature detected by the temperature sensor to the external device as the electric signal, by radio using the communication device on the circuit board 71.

Next, a method of adjusting a direction of the opening 20b of the oil gap 20a of the lubricant deterioration sensor 139b will be described. Hereinafter, the lubricant deterioration sensor 139b will be described, but the lubricant deterioration sensors other than the lubricant deterioration sensor 139b, such as the lubricant deterioration sensors 137a, 137b, and 139a, have the same configuration.

The external device of the lubricant deterioration sensor 139b can identify types and quantities of contaminant contained in the lubricant 131a of the reducer 131, based on a color detected by the RGB sensor 73. That is, the lubricant deterioration sensor 139b can detect a deterioration degree of the lubricant 131a by detecting the color of the contaminant contained in the lubricant 131a.

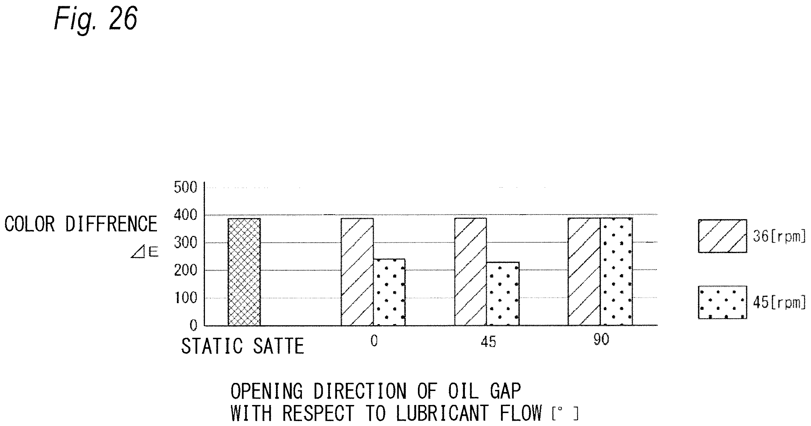

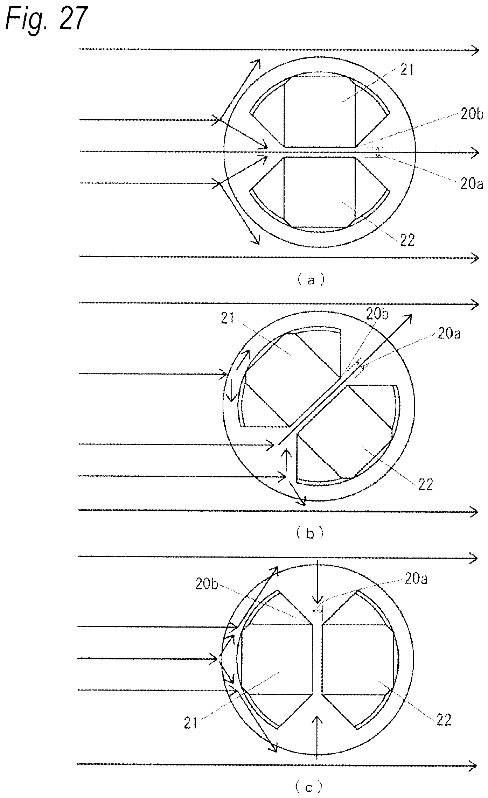

Fig. 26 is a view illustrating an example of a relationship between the direction of the opening 20b of the oil gap 20a with respect to flow of the lubricant 131a and a color difference ΔE of a color detected by the RGB sensor 73 with respect to the black color. Fig. 27(a) is a view illustrating a state where the direction of the opening 20b of the oil gap 20a with respect to the flow of the lubricant 131a represents 0°. Fig. 27(b) is a view illustrating a state where the direction of the opening 20b of the oil gap 20a with respect to the flow of the lubricant 131a represents 45°. Fig. 27(c) is a view illustrating a state where the direction of the opening 20b of the oil gap 20a with respect to the flow of the lubricant 131a represents 90°.

The color difference ΔE of the color detected by the RGB sensor 73 with respect to the black color can be calculated by using each RGB value of the color detected by the RGB sensor 73 and based on the following equation expressed in Expression 1.

[Expression 1]

$$\Delta E = \sqrt{R^2 + G^2 + B^2}$$

In an experiment which extracts the relationship illustrated in Fig. 26, new oil having lower deterioration degree is used as the lubricant 131a.

In addition, in Fig. 26, the term "static state" means a time when the flow of the lubricant 131a is stopped. When the flow of the lubricant 131a is stopped, the direction of the opening 20b of the oil gap 20a with respect to the flow of the lubricant 131a does not affect the color difference ΔE of the color detected by the RGB sensor 73 with respect to the black color. Therefore, the color difference ΔE of the color detected by the RGB sensor 73 with respect to the black color "static state" becomes a determination criteria for determining the relationship between the direction of the opening 20b of the oil gap 20a with respect to the flow of the lubricant 131a and the color difference ΔE of the color detected by the RGB sensor 73 with respect to the black color.

In Fig. 26, 36 rpm and 45 rpm represent that a rotation speed of the arm 113 with respect to the arm 112 is indicated by using the number of revolutions per one minute. Since the lubricant deterioration sensor 139b is attached to the arm 113, the rotation of the arm 113 with respect to the arm 112 causes the lubricant deterioration sensor 139b to move inside the lubricant 131a. That is, 36 rpm and 45 rpm indirectly represent the speed of the flow of the lubricant 131a with respect to the lubricant deterioration sensor 139b.

In Figs. 27(a) to 27(c), arrows other than an arrow representing the oil gap 20a indicate the flow of the lubricant 131a.

Accuracy in detecting the deterioration of the lubricant 131a can be determined by the color difference ΔE of the color detected by the RGB sensor 73 with respect to the black color. That is, in Fig. 26, the accuracy in detecting the deterioration of the lubricant 131a becomes poor when the number of revolutions of the arm 113 with respect to the arm 112 is 45 rpm, which is a case where the direction of the opening 20b of the oil gap 20a with respect to the flow of the lubricant 131a is 0° and 45°. As described above, in some cases, the accuracy in detecting the deterioration of the lubricant 131a becomes poor due to the direction of the opening 20b of the oil gap 20a with respect to the flow of the lubricant 131a.

In the lubricant deterioration sensor 139b, the direction of the opening 20b of the oil gap 20a can be adjusted.

First, the hexagon socket head cap bolt 11 is unfastened by using a jig inserted into the jig contact portion 11a so that the replaceable unit 60 can be rotated with respect to the main body 10.

Then, in a state where the jig contact portion 32 of the housing 30 is gripped by a jig so as to prevent the rotation of the housing 30 with respect to the arm 113, the replaceable unit 60 is rotated with respect to the housing 30 by using the jig inserted into the jig contact portion 91. If the replaceable unit 60 is rotated with respect to the housing 30, the fitting portion 83 of the unit body 80 of the replaceable unit 60 and the holder 40 to which the fitting portion 43 is fitted are rotated with respect to the housing 30. If the holder 40 is rotated with respect to the housing 30, the oil gap 20a formed in the gap forming member 20 supported by the holder 40 is rotated with respect to the housing 30. That is, the direction of the opening 20b of the oil gap 20a is changed by the rotation of the replaceable unit 60 with respect to the housing 30.

Lastly, the hexagon socket head cap bolt 11 is unfastened by using the jig which is inserted into the jig contact portion 11a so that the replaceable unit 60 cannot be rotated with respect to the housing 30.

As described above, each lubricant deterioration sensor such as the lubricant deterioration sensor 139b causes the RGB sensor 73 to detect a color with respect to light which is not absorbed by the contaminant contained in the lubricant 131a in the oil gap 20a, out of the white light emitted by the white LED 72. Accordingly, it is possible to immediately detect the color of the contaminant contained in the lubricant 131a of the reducer 131. That is, each lubricant deterioration sensor enables an external device such as a computer to immediately identify types and quantities of the contaminant contained in the lubricant 131a of the reducer 131, based on the color detected by the RGB sensor 73. Each lubricant deterioration sensor may be configured so that an electronic component which identifies the types and quantities of the contaminant contained in the lubricant, based on the color detected by the RGB sensor 73, is included in the electronic component group 70.

In general, locus accuracy of the arm in the industrial robot greatly depends on performance of the reducer used for the joints. Therefore, it is important to properly replace the reducer for the industrial robot when the performance becomes poor. However, when the reducer for the industrial robot is replaced, it is inevitable that the industrial robot including the reducer or an industrial robot-installed production line is to be stopped. For this reason, in order to accurately predict the time to replace the reducer for the industrial robot, it is very important that failure of the reducer for the industrial robot is predicted properly. Therefore, as described above, each lubricant deterioration sensor for the industrial robot 100 enables the external device such as the computer to immediately identify the types and quantities of the contaminant contained in the lubricant 131a of the reducer 131, based on the color detected by the RGB sensor 73. Accordingly, the industrial robot 100 and each reducer of the industrial robot 100 can immediately predict the failure.

In addition, in each lubricant deterioration sensor such as the lubricant deterioration sensor 139b, a consumable component such as the white LED 72 is included in the replaceable unit 60. Accordingly, it is possible to lengthen the life span by replacing the replaceable unit 60 from the main body 10.

As described above, it is very important that the failure of the reducer for the industrial robot is predicted properly. Therefore, in the industrial robot 100 and each reducer of the industrial robot 100, it is possible to lengthen the life span of each lubricant deterioration sensor which can immediately identify the types and quantities of the contaminant contained in the lubricant 131a. Accordingly, the accuracy in immediately predicting the failure can be maintained for a longer period of time.

In each lubricant deterioration sensor such as the lubricant deterioration sensor 139b, the consumable component such as the white LED 72 is unitized as the replaceable unit 60. Accordingly, the consumable component such as the white LED 72 is replaced by replacing the replaceable unit 60 from the main body 10. Therefore, each lubricant deterioration sensor such as the lubricant deterioration sensor 139b enables not only a manufacturer of the lubricant deterioration sensor but also a user of the lubricant deterioration sensor to easily replace the consumable component such as the white LED 72.

The hexagon socket head cap bolt 66 of the replaceable unit 60 is screwed into the screw hole 86a of the through-hole 86 of the replaceable unit 60 from the unit replacement opening 39 side of the main body 10, and comes into contact with the main body 10 on the opposite side to the unit replacement opening 39. In this manner, the replaceable unit 60 is separated from the main body 10 on the opposite side to the unit replacement opening 39. That is, in each lubricant deterioration sensor such as the lubricant deterioration sensor 139b, the replaceable unit 60 is easily detached from the main body 10 in such a way that the hexagon socket head cap bolt 66 of the replaceable unit 60 is screwed into the screw hole 86a of the through-hole 86 of the replaceable unit 60. Therefore, each lubricant deterioration sensor such as the lubricant deterioration sensor 139b can facilitate the replacement of the replaceable unit 60 from the main body 10.

In addition, in each lubricant deterioration sensor such as the lubricant deterioration sensor 139b, the replaceable unit 60 includes the hexagon socket head cap bolt 66 which is to be screwed into the screw hole 86a of the through-hole 86 so that the replaceable unit 60 is detached from the main body 10. Accordingly, it is not necessary to separately prepare a screw to be screwed into the screw hole 86a of the through-hole 86 in order to detach the replaceable unit 60 from the main body 10. Therefore, each lubricant deterioration sensor such as the lubricant deterioration sensor 139b can facilitate the replacement of the replaceable unit 60 from the main body 10.

In each lubricant deterioration sensor such as the lubricant deterioration sensor 139b, the button-type battery 74 whose life span is shorter than the life span of the white LED 72 can be replaced from the unit body 80. Accordingly, it is possible to lengthen the life span by replacing the button-type battery 74 from the unit body 80. In each lubricant deterioration sensor such as the lubricant deterioration sensor 139b, as long as the replaceable unit 60 can be replaced from the main body 10, the button-type battery 74 with the replaceable unit 60 can also be replaced from the main body 10. Accordingly, the button-type battery 74 may be configured so as not to be replaceable from the unit body 80.

The white LED 72 and the RGB sensor 73 are arranged on the opposite side to the battery replacement opening 87 in the cover 64 of the replaceable unit 60. In contrast, the button-type battery 74 is arranged on the battery replacement opening 87 side in the cover 64. That is, in each lubricant deterioration sensor such as the lubricant deterioration sensor 139b, when the button-type battery 74 is replaced from the unit body 80, as illustrated in Fig. 16, the cover 64 hides the white LED 72 and the RGB sensor 73 from a view of a worker. Therefore, in each lubricant deterioration sensor such as the lubricant deterioration sensor 139b, when the button-type battery 74 is replaced from the unit body 80, it is possible to minimize a possibility that the worker may touch the white LED 72 and the RGB sensor 73. That is, each lubricant deterioration sensor such as the lubricant deterioration sensor 139b can facilitate the worker's replacement of the button-type battery 74.

The replaceable battery according to the present invention is the button-type battery in the present embodiment. However, for example, a battery other than the button-type battery, such as a lithium-ion battery, may be employed.

In each lubricant deterioration sensor such as the lubricant deterioration sensor 139b, the holder 40 is arranged inside the screw 31 so as to be separate from the screw 31 so that a portion of the optical path 72a is arranged inside the screw 31. Accordingly, for example, when a screw hole such as the screw hole 113a is formed to be smaller than the screw 31, even when the screw 31 of the housing 30 is deformed inward by externally coming into contact with a screw hole of the machine body, such as the screw hole 113a of the arm 113, the deformation of the screw 31 is less likely to be transferred to the holder 40 arranged to be separate from the screw 31, and the optical path 72a is less likely to be changed due to the deformation of the holder 40. Therefore, in each lubricant deterioration sensor such as the lubricant deterioration sensor 139b, it is possible to suppress that accuracy in detecting the deterioration of the lubricant 131a in the machine body becomes poor when each lubricant deterioration sensor is fixed to the machine body by using the screw 31.

In each lubricant deterioration sensor such as the lubricant deterioration sensor 139b, the screw 31 comes into contact with the machine body by being screwed into the screw hole of th machine body. Consequently, the screw 31 is likely to be deformed inward. Therefore, there is a considerably advantageous effect in that it is possible to suppress that the accuracy in detecting the deterioration of the lubricant in the machine body becomes poor when the screw 31 is fixed to the machine body.

When the housing 30 includes a configuration of being fixed to the machine body such as the arm 113 without using the screw 31, a portion of the screw 31 illustrated in Fig. 3 may simply serve as a contact portion which externally comes into contact with the machine body. For example, in the housing 30, the portion of the screw 31 may be a simple cylindrical portion having no screw. In each lubricant deterioration sensor such as the lubricant deterioration sensor 139b, when the contact portion is a portion to be inserted into a hole of the machine body, the contact portion comes into contact with the machine body by being inserted into the hole of the machine body. Consequently, the contact portion is likely to be deformed inward. Accordingly, similar to the configuration in which the contact portion is the screw 31, there is a considerably advantageous effect in that it is possible to suppress that the accuracy in detecting the deterioration of the lubricant in the machine body becomes poor when the contact portion comes into contact with the machine body. In each lubricant deterioration sensor such as the lubricant deterioration sensor 139b, even when the contact portion comes into contact with machine body without being inserted into the hole of the machine body, when the contact portion is deformed inward by externally coming into contact with the machine body, the deformation of the screw 31 is less likely to be transferred to the holder 40 which is arranged to be separated from the contact portion. Accordingly, it is possible to suppress that the accuracy in detecting the deterioration of the lubricant in the machine body becomes poor when the contact portion comes into contact with the machine body.

In some cases, the lubricant 131a is obtained by adding various types of additive thereto, which include a friction reducing agent such as organic molybdenum (Mo) of molybdenum dithiocarbamate (MoDTC) or molybdenum dithiocarbamate (MoDTP) for decreasing friction on a friction surface, an extreme-pressure additive such as an SP-based additive for improving extreme-pressure lubricity representing the performance for suppressing the seizure of the friction surface, and a dispersing agent such as calcium sulfonate for suppressing generation and adhesion of sludge. These types of additive are separated from the lubricant 131a due to the deterioration of the lubricant 131a and in such a way that the additive adheres to, binds with or settles on a metal surface of the industrial robot 100 and the reducer. Based on the detected color, each lubricant deterioration sensor can identify not only an quantity of ion powder contained in the lubricant 131a but also a deterioration degree of base oil, and can identify an increase in the contaminant such as the sludge which may be caused by a decrease in various types of additive added to the lubricant 131a. Therefore, the industrial robot 100 and each reducer of the industrial robot 100 can improve the accuracy in predicting the failure as compared with a technique where the failure of the reducer is predicted based on only the density of the iron powder.

In addition, in each lubricant deterioration sensor such as the lubricant deterioration sensor 139b, the housing 30 rotatably supports the replaceable unit 60 so as to change the direction of the opening 20b of the oil gap 20a when the replaceable unit 60 is rotated. Accordingly, it is possible to adjust the direction of the opening 20b of the oil gap 20a when the housing 30 is fixed to the industrial robot 100, so as to improve the accuracy in detecting the deterioration of the lubricant 131a of the industrial robot 100 when the housing 30 is fixed to the industrial robot 100. Therefore, the industrial robot 100 and each reducer of the industrial robot 100 can predict the failure very accurately.

In addition, in each lubricant deterioration sensor such as the lubricant deterioration sensor 139b, the replaceable unit 60 includes the jig contact portion 91 serving as a portion in which the drive force of the rotation with respect to the housing 30 is received from the outside by using the contact force, at a position where the lubricant 131a is not touched when the housing 30 is fixed to the industrial robot 100. Therefore, in each lubricant deterioration sensor, it is possible to adjust the direction of the opening 20b of the oil gap 20a when the housing 30 is fixed to the industrial robot 100, so as to improve the accuracy in detecting the deterioration of the lubricant 131a of the industrial robot 100 after the housing 30 is fixed to the industrial robot 100.

In addition, in each lubricant deterioration sensor such as the lubricant deterioration sensor 139b, the hexagon socket head cap bolt 11 which prevents the rotation of the replaceable unit 60 with respect to the housing 30 by coming into contact with both the housing 30 and the replaceable unit 60 includes the jig contact portion 11a serving as a portion in which the drive force for coming into contact with both the housing 30 and the replaceable unit 60 is received from the outside by using the contact force, at a position where the lubricant 131a is not touched when the housing 30 is fixed to the industrial robot 100. Therefore, it is possible to fix the direction of the opening 20b of the oil gap 20a when the housing 30 is fixed to the industrial robot 100, so as to improve the accuracy in detecting the deterioration of the lubricant 131a of the industrial robot 100 after the housing 30 is fixed to the industrial robot 100.

In addition, each lubricant deterioration sensor is the white LED whose light emitting element emits the white light. Accordingly, for example, as compared to a configuration in which the light emitting element is a lamp other than an LED, each lubricant deterioration sensor can be downsized. Therefore, the industrial robot 100 and each reducer of the industrial robot 100 can be downsized. The light emitting element according to the present invention may be any configuration element other than an LED. For example, the light emitting element may be a lamp other than an LED. In addition, the light emitting element may include a red LED or a red light lamp other than an LED, a green LED or a green light lamp other than an LED, or a blue LED or a blue light lamp other than an LED. The configuration element may emit the white light by combining each color light emitted from LEDs or the lamps other than an LED.

In addition, each lubricant deterioration sensor is configured so that the light reflection surfaces 21b and 22b which bend the optical path 72a are formed in the gap forming member 20. Accordingly, as compared to a configuration in which the optical path 72a is formed linearly from the white LED 72 to the RGB sensor 73, the overall lubricant deterioration sensor can be downsized by arranging the white LED 72 and the RGB sensor 73 to be close to each other. In addition, in each lubricant deterioration sensor, the gap forming member 20 has not only a function of forming the oil gap 20a but also a function of bending the optical path 72a. Accordingly, as compared to a configuration in which a member for bending the optical path 72a is separately provided in place of the gap forming member 20, it is possible to reduce the number of components. Therefore, the industrial robot 100 and each reducer of the industrial robot 100 can be downsized, and can reduce the number of components therein.

In particular, in each lubricant deterioration sensor, the gap forming member 20 is configured to have the two right-angle prisms 21 and 22 respectively having the light reflection surfaces 21b and 22b which bend the optical path 72a by 90 degrees. Each lubricant deterioration sensor is configured so that the oil gap 20a is formed between the two right-angle prisms 21 and 22 by causing the light reflection surfaces 21b and 22b of the two right-angle prisms 21 and 22 to bend the optical path 72a by 180 degrees. Accordingly, each lubricant deterioration sensor can be downsized using a simple configuration having the reduced number of components. Therefore, the industrial robot 100 and each reducer of the industrial robot 100 can be downsized using the simple configuration having the reduced number of components.

In addition, each lubricant deterioration sensor includes the holder 40 which surrounds at least a portion of the optical path 72a, and is configured so that the surface of the holder 40 is subjected to treatment for preventing the light reflection. Accordingly, it is possible to prevent the RGB sensor 73 from receiving unnecessarily reflected light. For this reason, as compared to a configuration in which the RGB sensor 73 receives the unnecessarily reflected light, it is possible to improve the accuracy in detecting the color of the contaminant contained in the lubricant 131a. Therefore, the industrial robot 100 and each reducer of the industrial robot 100 can improve the accuracy in predicting the failure.

In addition, in each lubricant deterioration sensor, surfaces having the oil gap 20a within the gap forming member 20, that is, the light emitting surface 21c of the right-angle prism 21 and the light incident surface 22a of the right-angle prism 22 may be subjected to oil repelling treatment. In each lubricant deterioration sensor, when the light emitting surface 21c of the right-angle prism 21 and the light incident surface 22a of the right-angle prism 22 are subjected to the oil repelling treatment, the lubricant 131a is allowed to easily flow through the oil gap 20a. Accordingly, as compared to a configuration in which the lubricant 131a is likely to stay in the oil gap 20a, it is possible to improve the accuracy in detecting the color of the contaminant contained in the lubricant 131a. In addition, in each lubricant deterioration sensor, when the light emitting surface 21c of the right-angle prism 21 and the light incident surface 22a of the right-angle prism 22 are subjected to the oil repelling treatment, dirt is less likely to adhere to the light emitting surface 21c of the right-angle prism 21 and the light incident surface 22a of the right-angle prism 22. Accordingly, it is possible to suppress that the accuracy in detecting the color of the contaminant contained in the lubricant 131a becomes poor due to the adhering dust. Therefore, the industrial robot 100 and each reducer of the industrial robot 100 can improve the accuracy in predicting the failure.

The accuracy in predicting the failure of the reducer can be improved by combinedly using the lubricant deterioration sensor according to the present invention, a temperatures sensor for measuring the temperature of the lubricant, and a monitoring mechanism for monitoring a current value of a motor.

The right-angle prisms 21 and 22 of the gap forming member 20 are made of glass in the present embodiment. However, for example, materials other than the glass, such as silicone resin, may be used. A configuration can be made so that the dirt becomes less likely to adhere to the surface having the oil gap 20a by forming the right-angle prisms 21 and 22 using the silicone resin.

In addition, the gap forming member 20 is configured to have the two right-angle prisms 21 and 22 in the present embodiment, but may be configured to have three or more prisms.

In each lubricant deterioration sensor, the white LED 72 and the RGB sensor 73 may be arranged unlike in the arrangement described in the present embodiment. For example, in each lubricant deterioration sensor, the optical path 72a may be formed linearly from the white LED 72 to the RGB sensor 73.

In addition, in each lubricant deterioration sensor, the optical path 72a may be bent by a configuration excluding the right-angle prism.

In addition, as a method of communicating with the external device, each lubricant deterioration sensor employs radio communication using the incorporated communication device in the present embodiment. However, for example, wired communication may be employed.

In addition, as means for supplying electric power, each lubricant deterioration sensor employs the incorporated battery in the present embodiment. However, for example, a cable for electrically connecting the external power source and the lubricant deterioration sensor to each other may be employed.

A position for installing each lubricant deterioration sensor is not limited to the position illustrated in the present embodiment, but it is preferable to appropriately set the position to match an operating purpose of the industrial robot.

In addition, the machine according to the present invention is the reducer for the industrial robot or the industrial robot, but may be a machine other than the reducer for the industrial robot or the industrial robot.

Industrial Applicability

According to the present invention, it is possible to provide a lubricant deterioration sensor which can immediately identify types and quantities of contaminant contained in lubricant of a machine.

Reference Signs List

| | |
|---|---|
| 10 | main body |
| 20 | gap forming member |
| 20a | oil gap |
| 20b | opening |

| | |
|---|---|
| 30 | housing (fixing member) |
| 39 | unit replacement opening |
| 40 | holder (support member) |
| 43 | fitting portion (first fitting portion) |
| 60 | replaceable unit |
| 64 | cover |
| 66 | hexagon socket head cap bolt (detaching screw) |
| 72 | white LED (light emitting element) |
| 72 | a optical path |
| 73 | RGB sensor (color light receiving element) |
| 74 | button-type battery (replaceable battery) |
| 80 | unit body |
| 83 | fitting portion (second fitting portion) |
| 86 | through-hole |
| 86a | screw hole |
| 87 | battery replacement opening |
| 100 | industrial robot (machine) |
| 112, 113, 114, 115, 116 | arm |
| 120, 130 | joint |
| 131 | reducer (reducer for machine and industrial robot) |
| 131a | lubricant |
| 132 | reducer body (machine body) |
| 137a, 137b, 139a, 139b | lubricant deterioration sensor |
| 140, 150, 160, 170 | joint |

**Claims**

1. A lubricant deterioration sensor (139b) which is configured to be installed in a machine body (132) so as to detect deterioration of lubricant in the machine body (132), the sensor (139b) comprising:

   a main body (10) including a fixing member (30) configured to be fixed to the machine body (132); and
   a replaceable unit (60) that can be replaced from the main body (10),
   wherein the main body (10) includes a gap forming member (20) having an oil gap (20a) which the lubricant (131a) is allowed to enter,
   the replaceable unit (60) includes a light emitting element (72) configured to emit light and a color light receiving element (73) configured to detect a color of the received light,
   the gap forming member (20) is configured to allow the light emitted by the light emitting element (72) to pass therethrough,
   the oil gap (20a) is arranged on an optical path formed from the light emitting element (72) to the color light receiving element (73),
   the main body (10) has a first fitting portion (43), and the replaceable unit (60) has a second fitting portion (83), and
   the first fitting portion (43) and the second fitting portion (83) are fitted to each other in a state where a positional relationship between the gap forming member (20) and the light emitting element (72) and the color light receiving element (73) becomes a predetermined positional relationship,
   wherein the main body (10) includes a support member (40) which supports the gap forming member (20),
   the first fitting portion (43) is disposed in the support member (40), and the fixing member (30) rotatably supports the support member (40) and the replaceable unit (60) so that an opening direction of the oil gap (20a) varies when the support member (40) and the replaceable unit (60) are rotated.

2. The lubricant deterioration sensor (139b) according to Claim 1, wherein
   the main body (10) has a unit replacement opening (39) through which the replaceable unit (60) is allowed to pass when the replaceable unit (60) is detached from the main body (10),
   the replaceable unit (60) has a through-hole which causes the unit replacement opening side and an opposite side to the unit replacement opening (39) to communicate with each other, and
   at least a portion of the through-hole (86) is a screw hole (86a).

3. The lubricant deterioration sensor (139b) according to Claim 2, wherein

the replaceable unit (60) includes a detaching screw which is configured to come into contact with the main body (10) on the opposite side to the unit replacement opening (39) via the through-hole (86) screwed into the screw hole (86a) from the unit replacement opening side so that the replaceable unit (60) is separated from the main body (10) on the opposite side to the unit replacement opening (39).

4. The lubricant deterioration sensor according to Claim 1, wherein
the replaceable unit (60) includes a unit body (80) which supports the light emitting element (72) and the color light receiving element (73) and a replaceable battery (74) which can be replaced from the unit body, and
the replaceable battery (74) has a life span which is longer than a life span of the light emitting element (72), and may supply electric power to at least the light emitting element (72).

5. The lubricant deterioration sensor (139b) according to Claim 4, wherein
the replaceable unit (60) includes a cover (64) which covers the light emitting element (72) and the color light receiving element (73),
the unit body (80) has a battery replacement opening (87) through which the replaceable battery (74) is allowed to pass when the replaceable battery (74) is detached from the unit body (80),
the light emitting element (72) and the color light receiving element (73) are arranged on an opposite side to the battery replacement opening (87) in the cover (64), and
the replaceable battery (74) is arranged on the battery replacement opening side in the cover (64).

6. A machine (100) comprising the lubricant deterioration sensor (139b) and the machine body (132) according to any one of Claims 1 to 5.

7. The machine (100) according to Claim 6, wherein
the machine (100) is a reducer (132) for an industrial robot, and
the machine body (132) is a reducer body.

8. The machine (100) according to Claim 6, wherein
the machine (100) is an industrial robot, and
the machine body (132) includes an arm (112-116) and a reducer (131) which is used for a joint (140,150,160,170) of the arm (112-116), and
the lubricant (131a) is lubricant of the reducer (131).

**Patentansprüche**

1. Sensor (139b) für Verschleiß von Schmiermittel, der so eingerichtet ist, dass er in einem Vorrichtungs-Körper (132) installiert wird, um Verschleiß von Schmiermittel in dem Vorrichtungs-Körper (132) zu erfassen, wobei der Sensor (139b) umfasst:

einen Haupt-Körper (10), der ein Befestigungs-Element (30) enthält, das zum Befestigen an dem Vorrichtungs-Körper (132) eingerichtet ist; und
eine entnehmbare Einheit (60), die aus dem Haupt-Körper (10) entnommen werden kann,
wobei der Haupt-Körper (10) ein einen Spalt bildendes Element (20) enthält, das einen Ölspalt (20a) aufweist, in den das Schmiermittel (131a) eintreten kann,
wobei die entnehmbare Einheit (60) ein lichtemittierendes Element (72), das so eingerichtet ist, dass es Licht emittiert, sowie ein Element (73) zum Empfangen von Farblicht enthält, das so eingerichtet ist, dass es eine Farbe des empfangenen Lichtes erfasst,
das einen Spalt bildende Element (20) so eingerichtet ist, dass es das von dem lichtemittierenden Element (72) emittierte Licht hindurchtreten lässt,
der Ölspalt (20a) auf einem Lichtweg angeordnet ist, der von dem lichtemittierenden Element (72) zu dem Element (73) zum Empfangen von Farblicht ausgebildet ist,
der Haupt-Körper (10) einen ersten Pass-Abschnitt (43) aufweist und die entnehmbare Einheit (60) einen zweiten Pass-Abschnitt (83) aufweist, und
der erste Pass-Abschnitt (43) und der zweite Pass-Abschnitt (83) in einem Zustand ineinander gepasst werden, in dem eine Positionsbeziehung zwischen dem einen Spalt bildenden Element (20) und dem lichtemittierenden Element (72) sowie dem Element (73) zum Empfangen von Farblicht eine vorgegebene Positionsbeziehung wird,
der Haupt-Körper (10) ein Trage-Element (40) enthält, das das einen Spalt bildende Element (20) trägt,

der erste Pass-Abschnitt (43) in dem Trage-Element (40) angeordnet ist und das Befestigungs-Element (30) das Trage-Element (40) sowie die entnehmbare Einheit (60) drehbar so lagert, dass sich eine Öffnungsrichtung des Ölspalts (20a) ändert, wenn das Trage-Element (40) und die entnehmbare Einheit (60) gedreht werden.

2. Sensor (139b) für Verschleiß von Schmiermittel nach Anspruch 1, die weiter der Haupt-Körper (10) eine Öffnung (39) zum Entnehmen der Einheit aufweist, durch die die entnehmbare Einheit (60) hindurchtreten kann, wenn die entnehmbare Einheit (60) von dem Haupt-Körper (10) gelöst wird, die entnehmbare Einheit (60) ein Durchgangsloch aufweist, das bewirkt, dass die Seite der Öffnung zum Entnehmen der Einheit und eine der Öffnung (39) zum Entnehmen der Einheit gegenüberliegende Seite miteinander in Verbindung stehen, und wenigstens ein Abschnitt des Durchgangslochs (86) ein Gewindeloch (86a) ist.

3. Sensor (139b) für Verschleiß von Schmiermittel nach Anspruch 2, wobei die entnehmbare Einheit (60) eine Löse-Schraube enthält, die so eingerichtet ist, dass sie über das Durchgangsloch (86), das in das Gewindeloch (86a) von der Seite der Öffnungen zum Entnehmen der Einheit eingeschraubt ist, an der der Öffnung (39) zum Entnehmen der Einheit gegenüberliegenden Seite in Kontakt mit dem Haupt-Körper (10) kommt, so dass die entnehmbare Einheit (60) von dem Haupt-Körper (10) an der der Öffnung (39) zum Entnehmen der Einheit gegenüberliegenden Seite getrennt wird.

4. Sensor für Verschleiß von Schmiermittel nach Anspruch 1, wobei die entnehmbare Einheit (60) einen Körper (80) der Einheit enthält, der das lichtemittierende Element (72) und das Farblicht empfangende Element (73) sowie eine entnehmbare Batterie (74) aufnimmt, die aus dem Körper der Einheit entnommen werden kann, und die entnehmbare Batterie (70) eine Lebensdauer hat, die länger ist als die Lebensdauer des lichtemittierenden Elementes (72), und die wenigstens dem lichtemittierenden Element (72) Strom zuführen kann.

5. Sensor (139b) für Verschleiß von Schmiermittel nach Anspruch 4, wobei die entnehmbare Einheit (60) eine Abdeckung (64) enthält, die das lichtemittierende Element (72) und das Farblicht empfangende Element (73) abdeckt, der Körper (80) der Einheit eine Öffnung (87) zum Entnehmen der Batterie aufweist, durch die die entnehmbare Batterie (74) hindurchtreten kann, wenn die entnehmbare Batterie (74) von dem Körper (80) der Einheit gelöst wird, das lichtemittierende Element (72) und das Farblicht empfangende Element (73) an einer der Öffnung (87) zum Entnehmen der Batterie in der Abdeckung (64) gegenüberliegenden Seite angeordnet sind, und die entnehmbare Batterie (74) an der Seite der Öffnung zum Entnehmen der Batterie in der Abdeckung (64) angeordnet ist.

6. Vorrichtung (100), die den Sensor (139b) für Verschleiß von Schmiermittel sowie den Vorrichtungs-Körper (132) nach einem der Ansprüche 1 bis 5 umfasst.

7. Vorrichtung (100) nach Anspruch 6, wobei die Vorrichtung (100) ein Untersetzungsgetriebe (132) für einen Industrieroboter ist, und der Vorrichtungs-Körper (132) ein Untersetzungsgetriebe-Körper ist.

8. Vorrichtung (100) nach Anspruch 6, wobei die Vorrichtung (100) ein Industrieroboter ist, und der Vorrichtungs-Körper (132) einen Arm (112-116) sowie ein Untersetzungsgetriebe (131) enthält, das für ein Gelenk (140, 150, 160, 170) des Arms (112-116) eingesetzt wird, und das Schmiermittel (131a) ein Schmiermittel des Untersetzungsgetriebes (131) ist.


**Revendications**

1. Capteur de dégradation de lubrifiant (139b) configuré pour être installé dans un corps de machine (132) de manière à détecter la dégradation du lubrifiant dans le corps de machine (132), le capteur (139b) comprenant:

un corps principal (10) comprenant un élément de fixation (30) configuré pour se fixer au corps de machine (132); et
une unité remplaçable (60) pouvant être remplacée à partir du corps principal (10),
dans lequel le corps principal (10) comprend un formation d'orifice d'entrée (20) ayant un intervalle d'huile (20a) dans lequel le lubrifiant (131a) peut pénétrer,

l'unité remplaçable (60) comprend un élément émetteur de lumière (72) configuré pour émettre de la lumière et un élément récepteur de lumière colorée (73) configuré pour détecter une couleur de la lumière reçue,

l'élément de formation d'orifice d'entrée (20) est configuré pour permettre à la lumière émise par l'élément émettant de la lumière (72) de passer à travers celui-ci,

l'orifice d'entrée d'huile (20a) est agencé sur un trajet optique formé à partir de l'élément émetteur de lumière (72) vers l'élément récepteur de lumière de couleur (73),

le corps principal (10) présente une première partie d'ajustement (43) et l'unité remplaçable (60) présente une seconde partie d'ajustement (83), et

la première partie d'ajustement (43) et la seconde partie d'ajustement (83) sont adaptées l'une à l'autre dans un état dans lequel une relation de position entre l'élément de formation d'orifice d'entrée (20) et l'élément émetteur de lumière (72) et l'élément récepteur de lumière colorée (73) devient une relation de position prédéterminée,

dans lequel le corps principal (10) comprend un élément support (40) qui supporte l'élément de formation d'orifice d'entrée (20), la première partie d'ajustement (43) est disposée dans l'élément support (40), et l'élément de fixation (30) prend en charge de manière rotative l'élément support (40) et l'unité remplaçable (60) de sorte qu'une direction d'ouverture de l'orifice d'entrée d'huile (20a) varie lorsque tournent l'élément support (40) et l'unité remplaçable (60).

2. Capteur de dégradation de lubrifiant (139b) selon la revendication 1, dans lequel le corps principal (10) présente une ouverture de remplacement d'unité (39) à travers laquelle peut passer l'unité remplaçable (60) lorsque l'unité remplaçable (60) est séparée du corps principal (10),

l'unité remplaçable (60) présente un trou traversant permettant la communication entre le côté d'ouverture de remplacement d'unité et un côté opposé à l'ouverture de remplacement d'unité (39), et

au moins une partie du trou traversant (86) est un trou de vis (86a).

3. Capteur de dégradation de lubrifiant (139b) selon la revendication 2, dans lequel l'unité remplaçable (60) comprend une vis de séparation configurée pour venir au contact du corps principal (10) du côté opposé à l'ouverture de remplacement d'unité (39) par le trou traversant (86) vissé dans le trou de vis (86a) à partir du côté ouverture de remplacement d'unité de sorte que l'unité remplaçable (60) est séparée du corps de partie principale (10) du côté opposé à l'ouverture de remplacement de l'unité (39).

4. Capteur de dégradation de lubrifiant selon la revendication 1, dans lequel l'unité remplaçable (60) comprend un corps d'unité (80) qui prend en charge l'élément émetteur de lumière (72) et l'élément récepteur de lumière colorée (73) et une batterie remplaçable (74) qui peut être remplacée à partir du corps d'unité, et la batterie remplaçable (74) a une durée de vie supérieure à celle de l'élément émetteur de lumière (72) et peut alimenter au moins ledit élément émetteur de lumière (72).

5. Capteur de dégradation de lubrifiant (139b) selon la revendication 4, dans lequel l'unité remplaçable (60) comprend un couvercle (64) qui recouvre l'élément émetteur de lumière (72) et l'élément récepteur de lumière de couleur (73),

le corps principal (80) présente une ouverture de remplacement de batterie (87) à travers laquelle peut passer la batterie remplaçable (74) lorsque la batterie remplaçable (74) est séparée du corps principal (80),

l'élément émetteur de lumière (72) et l'élément récepteur de lumière de couleur (73) sont disposés sur un côté opposé à l'ouverture de remplacement de batterie (87) dans le couvercle (64), et

la batterie remplaçable (74) est disposée du côté de l'ouverture de remplacement de la batterie dans le couvercle (64).

6. Machine (100) comprenant le capteur de dégradation de lubrifiant (139b) et le corps de machine (132) selon l'une quelconque des revendications 1 à 5.

7. Machine (100) selon la revendication 6, dans laquelle la machine (100) est un réducteur (132) pour robot industriel, et le corps de la machine (132) est un corps réducteur.

8. Machine (100) selon la revendication 6, dans laquelle la machine (100) est un robot industriel, et le corps de machine (132) comprend un bras (112-116) et un réducteur (131) utilisé pour un joint (140, 150, 160, 170) du bras (112-116), et le lubrifiant (131a) est le lubrifiant du réducteur (131).

Fig. 1

# Fig. 2

*Fig. 3*

## Fig. 4

*Fig. 5*

## Fig. 6

( a )

( b )

Fig. 7

(a)

(b)

*Fig. 8*

( a )

( b )

*Fig. 9*

(a)

(b)

*Fig. 10*

40

42 — — 41

( a )

43

40

45

44

42 — — 41
42a — — 41a

( b )

*Fig. 11*

40

41

41a

( a )

40

42

42a

( b )

*Fig. 12*

40

43

45

44

( a )

40

42a    41a

42    41

45    44

42a    41a

( b )

Fig. 13

*Fig. 14*

( a )

( b )

*Fig. 15*

( a )

( b )

*Fig. 16*

*Fig. 17*

80

( a )

87    80

84    84

85    85

82
83

81

( b )

*Fig. 18*

(a)

(b)

*Fig. 19*

( a )

( b )

*Fig. 20*

*Fig. 21*

90

91 92 II

93

II

92

92

92

93

92

(a)

90

92

93

92 92

93

92

(b)

*Fig. 22*

90

(a)

90

93          91          92

(b)

Fig. 23

Fig. 24

*Fig. 25*

Fig. 26

EP 2 848 917 B1

EP 2 848 917 B1

*Fig. 27*

( a )

( b )

( c )

45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010045989 A1
- US 2011249257 A1

- JP 7146233 A
- JP 10104160 A

**Non-patent literature cited in the description**

- METHOD OF DISCRIMINATING HUE OF CONTAMINANT CONTAINED IN LUBRICANT. **TOMOHIKO YAMAGUCHI.** Faculty of Engineering, Research Report. Fukui University, March 2003, vol. 51, 81-88

- **TOMOMI HONDA.** TECHNOLOGY FOR DIAGNOSING AND INSPECTING DETERIORATION OF LUBRICANT. *Journal of Japan Society for Precision Engineering,* 2009, vol. 75 (3), 359-362